# FASCICULE DE BREVET EUROPEEN

(11) **EP 0 486 386 B1**
(45) Date de publication et mention de la délivrance du brevet: **17.07.1996**
(21) Numéro de dépôt: 91403044.0
(22) Date de dépôt: 14.11.1991
(51) Int. Cl.: C07D 207/16, A61K 31/40, C07D 405/06, C07D 491/08, C07D 207/24

(54) **Nouveaux dérivés de la N-benzoyl proline, leur procédé de préparation et les compositions pharmaceutiques qui les contiennent**
Neue N-Benzoylprolin-Derivate, Verfahren zur Herstellung und diese enthaltende Arzneimittel
Novel derivatives of N-benzoylproline, process for their preparation and drugs containing them

(30) Priorité: 14.11.1990 FR 9014087
(43) Date de publication de la demande: 20.05.1992
(73) Titulaire: ADIR ET COMPAGNIE, F-92415 Courbevoie Cédex (FR)
(72) Inventeur: Rault, Sylvain, F-14370 Moult (FR); Foloppe, Marie-Paule, F-61120 Vimoutiers (FR); Robba, Max, F-75004 Paris (FR); Boulouard, Michel, F-14000 Caen (FR); Renard, Pierre, F-78000 Versailles (FR); Devissaguet, Michelle, F-92200 Neuilly sur Seine (FR); Adam, Gérard, F-78600 Le Mesnil le Roi (FR)

(56) Documents cités:
- EP-A- 0 172 458
- EP-A- 0 238 319
- EP-A- 0 333 522
- EP-A- 0 345 428
- US-A- 3 524 849
- US-A- 3 763 183
- US-A- 4 123 544
- CHEMICAL ABSTRACTS, vol. 111, 1989, Columbus, Ohio, US; abstract no. 96934, M. R. PENA ET. AL.: '"A total synthesis of anthramycin" & J. Am. Chem. Soc. 1989, 111(14), 5417-24' page 712 ;
- CHEMICAL ABSTRACTS, vol. 99, 1983, Columbus, Ohio, US; abstract no. 87975, ANDERSON W. K. ET. AL.: 'Synthesis, evaluation of chemical reactivity, and murine antineoplastic activity of 2-hydroxy-5-(3,4-dich lorophenyl)-6,7- bis(hydroxymethyl)-2,3-dihydro-1 H-pyrrolizine-bis(2-propylcarbamate) and 2- acyloxy derivatives as potential water soluble prodrugs. & J. Med. Chem. 1983, 26(9), 1333-8' page 549 ;
- CHEMICAL ABSTRACTS, vol. 82, 1975, Columbus, Ohio, US; abstract no. 31358, KARIYONE KAZUO: '1H-Pyrrolo[2,1-c][1,4]benzodiazepines & JP-A-74016439 (Fujisawa) 22 Avril 1974' page 504 ;

## Description

La présente invention concerne de nouveaux dérivés de la N benzoyl proline, leur procédé de préparation et les compositions pharmaceutiques qui les contiennent.

De très nombreux dérivés de la N benzoyl proline sont connus Parmi eux un grand nombre est décrit en tant que simples imtermédiaires de synthèse de benzodiazépines à activité antiinflammatoire, analgésique, dépressive du système nerveux central, anti-cancéreuse, antiphage, antibactérienne : brevets US 3,763,183 ; 3,524,849 ; 4,427,587. Le brevet US 4,912,231 décrit des dérivés de la N benzoyl 4-hydroxy proline en tant qu'intermédiaires de synthèse.

Le brevet US 4,123,544 décrit des dérivés de la N benzoyl proline qui sont également des dérivés de l'acide acétyl salicylique et de ce fait revendiqués pour leurs propriétés analgésiques.

La publication J. Med. Chem 1983, 26, 9, 1333-1338 décrit la 1-(3,4-dichlorobenzoyl)-4-hydroxy trans L proline et son dérivé lactonique en tant qu'intermédiaire de synthèse de dérivés anticancéreux.

Le brevet US 3,896,149 décrit des dérivés de la N-benzoyl proline en tant qu'antiulcéreux.

Les brevets EP-A-0 172 458, EP-A-0 345 428 et EP-A-0 238 319 divulguent des dérivés de la proline qui sont des inhibiteurs de la prolyl-endopeptidase.

La demanderesse a présentement découvert de nouveaux dérivés de la N benzoyl proline doués d'activité antiamnésique très puissante.

Plus particulièrement la présente invention concerne des dérivés de formule générale (I) : 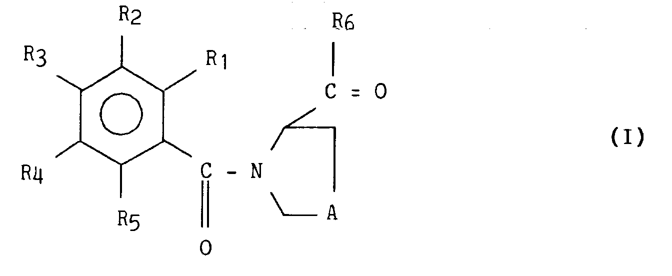
avec A représentant un groupement CO, ou CHOH,
R₁, R₂, R₃, R₄, R₅ identiques ou différents représentant indépendamment l'un de l'autre un atome d'hydrogène, de chlore ou de fluor, un groupement alkyle inférieur, cycloalkyle de 3 à 8 atomes de carbone, un groupement hydroxy, un groupement alkoxy inférieur, un groupement alkyle inférieur amino, un groupement dialkyl inférieur amino, un groupement phénylalkyle inférieur, un groupement phénylalkyle inférieur oxy, un groupement alkyle inférieur substitué par un ou plusieurs atomes d'halogène, ou (et) encore deux groupements adjacents parmi R₁, R₂, R₃, R₄, R₅ c'est à dire R₁ et R₂, ou R₂ et R₃ ou R₃ et R₄ ou R₄ et R₅ forment ensemble un pont -O-CH₂-O-, -O-CH₂-CH₂-O-, ou -O-CH=CH-O- étant entendu que :
. R₁, R₂, R₃, R₄, R₅ ne peuvent représenter simultanément un atome d'hydrogène,
. A ne peut représenter un groupement C=O que lorsque R₁ et R₂ ou R₂ et R₃ ou R₃ et R₄ ou R₄ et R₅ forment ensemble un pont -O-CH₂-O- ou -O-CH₂-CH₂-O- ou -O-CH=CH-O-,
   R₆ représente un groupement hydroxy ou un groupement alkoxy inférieur, un groupement amino ou NR₇R₈ ou -O-B-NR₇R₈ avec B alkyle inférieur et R₇, R₈ identiques ou différents représentant un atome d'hydrogène, un groupement alkyle inférieur, ou cycloalkyle ou cycloalkyle alkyle inférieur, phényle, phénylalkyle inférieur, phényle substitué ou phénylalkyle inférieur substitué, ou R₇ et R₈ forment avec l'azote qui les porte un système hétérocyclique mono, ou bicyclique, chaque cycle comprenant de cinq à six sommets et intégrant éventuellement dans son squelette de un à deux hétéroatomes choisi parmi azote, oxygène ou soufre et éventuellement substitué par un groupement alkyle inférieur ou phényle ou phényle alkyle inférieur, ou phénylalkyle alkyle inférieur substitué ou phényle substitué, le terme substitué affectant les termes phényle et phényle alkyle inférieur signifiant que le noyau aromatique des ces groupes peut être substitué par un ou plusieurs groupements alkyle inférieur, alkoxy inférieur, ou trifluorométhyle ou bien encore R₆ forme avec A un système lactonique interne, ce qui confère aux dérivés de formule (I) la structure particulière (I') : 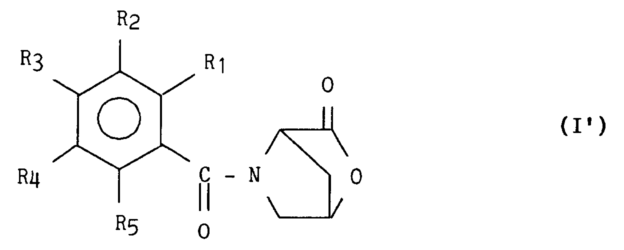 où R₁, R₂, R₃, R₄, R₅ ont la même définition que précedemment
   aux conditions que :
. si R₆ représente un groupement hydroxy et A représente un groupement CHOH ou R₆ et A forment ensemble un groupement lactonique interne (dérivés de formule (I')) on ne peut avoir simultanément R₁, R₄ et R₅ représentant chacun simultanément un atome d'hydrogène et R₂ et R₃ représentant chacun un atome de chlore, et symétriquement, R₅, R₁ et R₂ représentant un atome d'hydrogène et R₃ et R₄ représentant chacun un atome de chlore,
. si R₆ et A forment ensemble un groupement lactonique interne (dérivés de formule (I')) au moins deux groupements parmi R₁, R₂, R₃, R₄ et R₅ sont différents de l'atome d'hydrogène,
. R₁ ou R₅ ne peuvent représenter un groupement acétyloxy si simultanément R₂, R₃, R₄, R₆ et l'autre groupement parmi R₁ et R₅ représentent un atome d'hydrogène,
. étant entendu que par groupement cycloalkyle on entend des groupements de 3 à 8 atomes de carbone et que par groupement alkyle inférieur on entend des groupements alkyle linéaires ou ramifiés de 1 à 6 atomes de carbone,
ainsi que le cas échéant leurs isomères, épimères, diastéréoisomères et leurs sels d'addition à un acide ou une base pharmaceutiquement acceptable.

Parmi les acides que l'on peut utiliser pour salifier les composés de formule générale (I), on peut citer à titre non limitatif, les acides chlorhydrique, sulfurique, phosphorique, tartrique, malique, maléique, fumarique, oxalique, méthane-sulfurique, éthane-sulfurique, camphorique, citrique et...

Parmi les bases que l'on peut utiliser pour salifier les composés de formule générale (I), on peut citer à titre non limitatif, les hydroxydes de sodium, potassium, calcium, ou des bases organiques comme la diéthylamine, la benzylamine, la dicyclohexylamine, l'arginine, ou des carbonates de métaux alcalins ou alcalino-terreux.

L'invention s'étend aussi au procédé d'obtention des composés de formule générale (I) caractérisé en ce que l'on utilise comme matière première un dérivé de formule (II) : 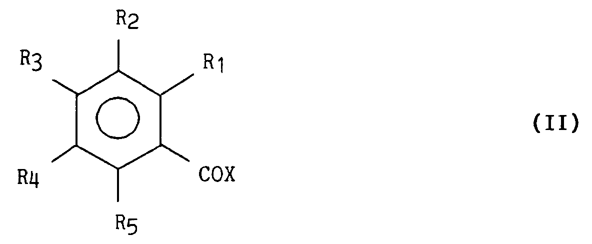
dans laquelle R₁, R₂, R₃, R₄ et R₅ ont la même définition que dans la formule (I) et X représente un atome d'halogène,
que l'on traite par le dérivé de formule (III) : 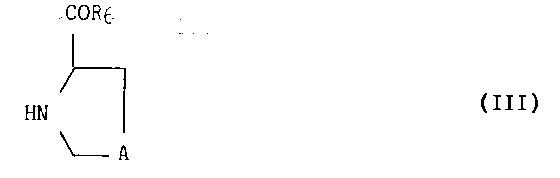
dans laquelle R₆ a la même définition que dans la formule (I),
pour conduire à un dérivé de formule (I) : 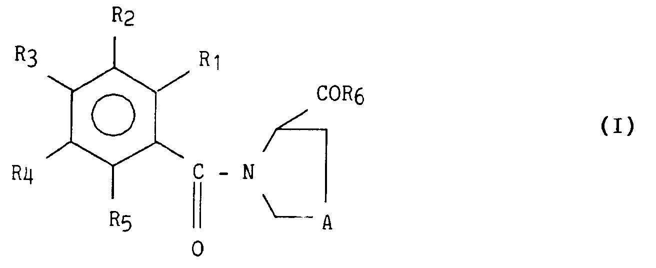
que l'on sépare le cas échéant en ses isomères et que l'on salifie, si on le désire, par un acide ou le cas échéant une base pharmaceutiquement acceptable,
dérivé de formule (I)qui lorsque R₆ représente un groupement OH peut être traité le cas échéant, après éventuelle activation de la fonction acide carboxylique,
- ou bien par une amine de formule NR₇R₈ ou bien par un dérivé de formule X-B-NR₇R₈ où B, R₇ et R₈ ont la même signification que dans la formule (I), et X représente un atome d'halogène, ou bien par un alcool aliphatique inférieur de formule R'₆OH où R'₆ représente un groupement alkyle inférieur pour obtenir un composé de formule (I) pour lesquels R₆ représente respectivement un groupement NR₇R₈ ou un groupement alkyle inférieur,
- ou bien par l'imidazole pour obtenir un dérivé de formule (I'),
dérivé de formule (I) ou (I') que l'on purifie si on le désire, que l'on sépare le cas échéant en ses isomères et que l'on salifie si on le désire, par un acide ou une base pharmaceutiquement acceptable.

Un cas particulier concerne les dérivés de l'invention pour lesquels A représente un groupement C=O. De tels dérivés peuvent être avantageusement obtenus en traitant un dérivé de formule (I/B) : 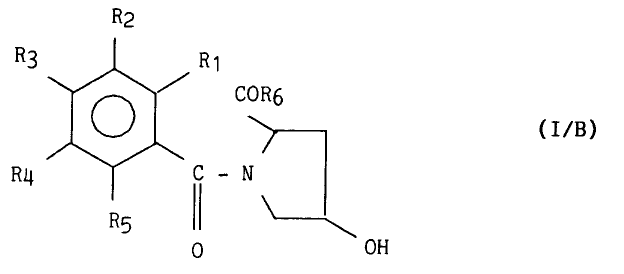
cas particulier des dérivés de formule (I) pour lesquels R₁, R₂, R₃, R₄, R₅ et R₆ ont la même définition que dans la formule (I) et A représente un groupement CHOH,
par un agent oxydant pour obtenir un dérivé de formule (I/C) : 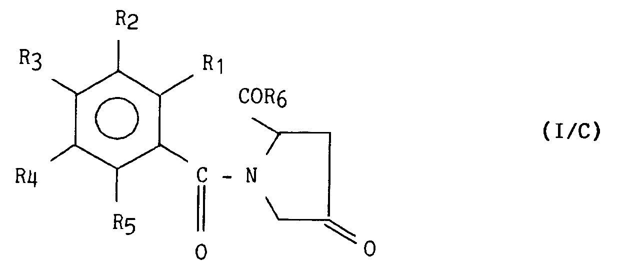
dans laquelle R₁, R₂, R₃, R₄, R₅ et R₆ ont la même définition que dans la formule (I),
que l'on purifie et que l'on salifie si on le désire, le cas échéant par un acide ou une base pharmaceutiquement acceptable,
et que l'on peut traiter, lorsque R₆ représente un groupement hydroxyle, en fonction d'un dérivé de formule (I) que l'on souhaite obtenir ou bien par une amine de formule NR₇R₈ ou par un dérivé de formule X-B-NR₇R₈ où B, R₇ et R₈ ont la même signification que dans la formule (I) et X représente un atome d'halogène, ou par un alcool aliphatique inférieur R'₆OH où R'₆ représnete un groupement alkyle inférieur pour obtenir un composé de formule (I) pour lesquels R₆ représente respectivement un groupement NR₇R₈ ou un groupement alkyle inférieur,
que l'on purifie si nécessaire et dont on sépare la cas échéant les isomères et que l'on salifie, si on le désire, par un acide ou une base pharmaceutiquement acceptable.

Les composés de formule (I) possèdent des propriétés pharmacologiques intéressantes.

L'étude pharmacologique des dérivés de l'invention a montré qu'ils étaient peu toxiques et antagonisent très fortement l'amnésie induite par la scopolamine à des doses très faibles.

Les composés de l'invention trouvent donc leur application dans le traitement des maladies résultant de troubles hypoxiques ischémiques et oxyprives et sont donc particulièrement indiqués pour améliorer les symptômes du déficit intellectuel, la pathologie du sujet âgé (maladie d'Alzheimer et troubles mnésiques en général), les troubles de l'attention, dans le traitement des infarctus cérébraux constitués et dans les vertiges d'origine centrale.

Les dérivés de l'invention dont les propriétés sont apparues les plus intéressantes sont ceux pour lesquels A représente un groupement CHOH.

La présente invention a également pour objet les compositions pharmaceutiques renfermant comme principe actif au moins un composé de formule générale (I) ou un de ses sels d'addition à un acide ou une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, paquets, les gélules, glossettes, tablettes, suppositoires, crèmes, pommades, gels dermiques, solutés injectables etc...

La posologie varie selon l'âge et le poids du patient, la nature et la sévérité de l'affection ainsi que la voie d'administration. Celle-ci peut être orale, nasale, rectale ou parentérale.

D'une manière générale la posologie unitaire s'échelonne entre 0,1 et 500 mg et peut être administrée 1 à 3 fois par jour.

Les exemples suivants illustrent l'invention et ne la limitent en aucune façon.

Les spectres de résonance magnétique nucléaire ¹H ont été réalisés en utilisant le TMS (tétraméthylsilane) comme référence interne. Les déplacements chimiques sont exprimés en partie par million (p.p.m.).

Les spectres infrarouge ont été effectués sous forme de pastille de bromure de potassium renfermant environ 1% du produit à analyser.

Les exemples ci-dessous pour lesquels A représente un groupement CHOH ont la formule générale ci-dessous : 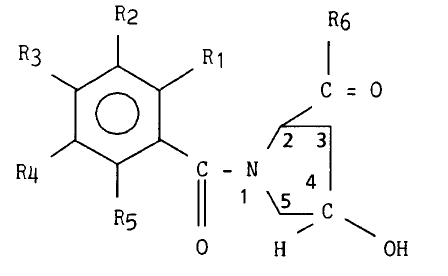
Les atomes de carbone numérotés 2 et 4 sont asymétriques et il existe donc de ces dérivés deux couples d'énantiomères :
TRANS-(L)- ou 2S, 4R
TRANS-(D)- ou 2R, 4S
CIS-(L)- ou 2S, 4S
CIS-(D) ou 2R, 4R.

Les exemples ci-dessous pour lesquels A représente un groupement CHOH ont été synthétisés à partir de la Trans-4-hydroxy-L- proline ou acide [(2S,4R)-(-)-4-hydroxy-2-pyrrolidine] carboxylique. Cependant les autres composés obtenus en partant :
- de la Trans-4-Hydroxy-D-proline ou acide [(2R,4S)-(+)-4-hydroxy-2-pyrrolidine] carboxylique,
- de la Cis-4-hydroxy-D-proline ou acide [(2R,4R)-(+)-4-hydroxy-2-pyrrolidine] carboxylique,
- de la Cis-4-hydroxy-L-proline ou acide [(2S,4S)-(-)-4-hydroxy-2-pyrrolidine] carboxylique,
font partie de l'invention au même titre que les précédents.

Lorsque A représente un groupement CH₂ ou un groupement CO, il existe deux isomères appelés L ou D (R ou S). Les exemples correspondants ci-dessous ont été réalisés à partir de la (L)proline ou de ces dérivés. Les deux séries d'isomères font néanmoins partie intégrante de l'invention.

Les préparations ne font pas partie de l'invention mais sont utiles pour réaliser la synthèse des dérivés de l'invention.

### PREPARATION 1 : CHLORURE DE 4,5-METHYLENE DIOXY 2-NITRO BENZOYLE

### STADE A : ACIDE 4,5-METHYLENE DIOXY 2-NITROBENZOIQUE

30 g (0, 18 mole) d'acide pipéronylique sont ajoutés avec précaution à 200 ml d'acide nitrique (d=1,42). Après une première addition de 5 g, la réaction est amorcée en chauffant et en agitant vigoureusement le mélange réactionnel à 50°C. L'addition des 25 g restants est réalisée par petites fractions. A la fin de l'addition, le mélange réactionnel est laissé sous agitation pendant 2 heures. Ajouter alors 200 ml d'eau, le produit de réaction insoluble est essoré, lavé avec 100 ml d'eau puis dissous dans une solution aqueuse saturée de bicarbonate de sodium. Eliminer l'insoluble. Acidifier le filtrat à 0°C et recueillir le précipité qui est essoré, lavé à l'eau et séché.
Rendement : 67%
Point de fusion : 172°C
Caractéristiques spectrales :
v CO 1710 cm⁻¹
v NO₂ 1515 et 1335 cm⁻¹

### STADE B : CHLORURE DE 4,5-METHYLENE DIOXY 2-NITRO BENZOYLE

5 g (0,23 mole) d'acide 4,5-méthylènedioxy 2-nitro benzoique sont mis en suspension dans 50 ml de chlorure de thionyle et chauffés au reflux pendant 2 heures. La solution obtenue est concentrée sous pression réduite. L'huile résiduelle cristallise dès l'addition de 50 ml d'éther de pétrole. Les cristaux sont essorés et recristallisés dans l'éther.
Rendement : 90%
Point de fusion : 70°C
Caractéristiques spectrales :
Infrarouge :
v C = O : 1775 cm⁻¹

| Composition centésimale : | | | | |
|---|---|---|---|---|
| Calculée | C: 41,85 | H: 1,75 | N: 6,10 | N: 15,44 |
| Trouvée | C: 41,78 | H: 1,67 | N: 5,94 | N: 15,32 |

### PREPARATION 2 : CHLORURE DE 3,4-DIMETHOXY BENZOYLE

20 g (0,1 mole) de l'acide 3,4 diméthoxy benzoïque sont mis en suspension dans 170 ml de chlorure de thionyle et chauffés au reflux pendant 2 heures. La solution est concentrée sous pression réduite. L'huile résiduelle cristallise dès l'addition d'éther de pétrole. Les cristaux sont essorés et lavés à l'éther de pétrole.
Point de fusion : 70°C
Caractéristiques spectrales :
Infrarouge :
v C = O : 1760 cm⁻¹

### PREPARATION 3 : CHLORURE DE 3,4-METHYLENE DIOXY BENZOYLE

20 g (0,12 mole) d'acide pipéronylique sont mis en suspension dans 170 ml de chlorure de thionyle et chauffés au reflux pendant 2 heures. La solution est concentrée sous pression réduite. L'huile résiduelle cristallise dès l'addition d'éther de pétrole. Les cristaux sont essorés et lavés à l'éther de pétrole.
Point de fusion : 80°C
Caractéristiques spectrales :
Infrarouge :
v C = O : 1750 cm⁻¹

### PREPARATION 4 : CHLORURE DE 3,4,5-TRIMETHOXY BENZOYLE

10 g (0,047 mole) d'acide 3,4,5-triméthoxy benzoïque sont mis en suspension dans 120 ml de chlorure de thionyle et chauffés au reflux pendant 2 heures. La solution est concentrée sous pression réduite. L'huile résiduelle cristallise dès l'addition d'éther de prétrole. Les cristaux sont essorés et lavés à l'éther de pétrole.
Point de fusion : 80°C
Caractéristiques spectrales :
Infrarouge
v C = O : 1750 cm⁻¹

### PREPARATION 5 : CHLORURE DE 4-HYDROXY BENZOYLE

Placer 10 g (0,072 mole) d'acide 4-hydroxy benzoïque dans 100 cm³ de toluène. Ajouter 20 g de pentachlorure de phosphore par petites portions et agiter sous léger chauffage pendant deux heures. Concentrer sous pression réduite. Triturer le résidu dans l'éther de pétrole. Essorer. Sécher. Laver à l'eau. Sécher.
On obtient de même : le chlorure de dihydroxy-3,4 benzoyle à partir d'acide 3,4-dihydroxy benzoïque.

### PREPARATION 6 CHLORURE DE 3,4-ETHYLENEDIOXY BENZOYLE

### STADE A : ACIDE 3,4-ETHYLENEDIOXY BENZOIQUE

10 g (0,06 mole) de 1,4-benzodioxanne 6-carboxaldéhyde dans 250 ml d'eau sont chauffés à 70-80°C à l'aide d'un bain-marie. Dans cette émulsion, on additionne goutte à goutte en 45 minutes une solution de 13,4 g (0,084 mole) de permanganate de potassium en solution dans 300 ml d'eau. Après cette addition l'agitation et le chauffage sont maintenus pendant 1 heure.
On ajoute ensuite de la potasse à 10% en quantité suffisante pour rendre la solution alcaline. Le mélange encore chaud est filtré, le bioxyde de manganèse formé est lavé 3 fois avec 50 ml d'eau. Le filtrat et les eaux de lavage sont réunis puis acidifiés au moyen d'acide chlorhydrique 12N. Le précipité blanc formé est essoré et lavé à l'eau et séché.
Rendement : 76%
Point de fusion : 142°C
Caractéristiques spectrales :
v CO : 1670 cm⁻¹

### STADE B : CHLORURE DE 3,4 ETHYLENEDIOXY BENZOYLE

En procédant comme dans le stade B de la préparation 1, mais en remplaçant l'acide pipéronylique par l'acide 3,4-éthylènedioxy benzoïque formé au stade précédent, on obtient le produit du titre.
Point de fusion : 103°C

### PREPARATION 7 : CHLORURE DE 4-TRIFLUOROMETHYL BENZOYLE

En procédant comme dans la préparation 1, stade B, mais en remplaçant l'acide 4,5-méthylènedioxy 2-nitro benzoïque par l'acide 4-trifluorométhyl benzoïque, on obtient le produit du titre.

### PREPARATION 8 : CHLORURE DE 4-CHLOROBENZOYLE

En procédant comme dans la préparation 1, stade B, mais en remplaçant l'acide 4,5-méthylènedioxy 2-nitro benzoïque par l'acide 4-chloro benzoïque, on obtient le produit du titre.

### PREPARATION 9 : CHLORURE DE 2-BENZYLE BENZOYLE

En procédant comme dans la préparation 1, stade B, mais en remplaçant l'acide 4,5-méthylènedioxy 2-nitro benzoïque par l'acide 2-benzyl benzoïque, on obtient le produit du titre.

### PREPARATION 10 : CHLORURE DE 4-BENZYLOXY BENZOYLE

En procédant comme dans la préparation 1, stade B, mais en remplaçant l'acide 4,5-méthylènedioxy 2-nitro benzoïque par l'acide 4-benzyloxy benzoïque, on obtient le produit du titre.

### PREPARATION 11 : CHLORURE DE 3,5 DITERT.BUTYL 4-HYDROXY BENZOYLE

En procédant comme dans la préparation 5, mais en remplaçant l'acide 4,5-méthylènedioxy 2-nitro benzoïque par l'acide 3,5 ditert.butyl 4-hydroxy benzoïque, on obtient le produit du titre.

### PREPARATION 12 : CHLORURE DE 6-BENZO [1,4]DIOXINE CARBONYLE

En procédant comme dans la préparation 5, mais en remplaçant l'acide 4,5-méthylènedioxy 2-nitro benzoïque par l'acide 6-benzo [1,4]dioxine carboxylique, on obtient le produit du titre.

### PREPARATION 13 : CHLORURE DE 2,3-METHYLENEDIOXY BENZOYLE

En procédant comme dans la préparation 1 - stade B, mais en remplaçant l'acide 4,5-méthylènedioxy 2-nitro benzoïque par l'acide 2,3-méthylène dioxy benzoïque, on obtient le produit du titre.

### PREPARATION 14 : CHLORURE DE 2,3-ETHYLENEDIOXY BENZOYLE

En procédant comme dans la préparation 2 - stade B, mais en remplaçant l'acide 4,5-méthylènedioxy 2-nitro benzoïque par l'acide 2,3-éthylène dioxy benzoïque, on obtient le produit du titre.

### PREPARATION 15 : CHLORURE DE 4-METHOXY BENZOYLE

A 10 g (0,06 mole) de l'acide 4-méthoxy benzoïque en suspension dans 60 ml d'éther de pétrole on additionne 2 équivalents de chlorure de thionyle et 0,1 g de chlorure d'aluminium. Ce mélange réactionnel est chauffé au reflux pendant 5 heures. La solution est concentrée sous pression réduite. L'huile résiduelle cristallise en présence d'éther de pétrole à 0°C.
Point de fusion : 24°C
Caractéristiques spectrales :
Infrarouge :
Bande C=O à 1770 cm⁻¹

### PREPARATION 16 : ACIDE 4-HYDROXY 1-(4,5-METHYLENEDIOXY 2-NITRO BENZOYL) PYRROLIDINE 2-CARBOXYLIQUE (FORME TRANS)

Dans un ballon tricol de 2 litres muni de 2 ampoules à brome et d'un thermomètre, refroidir par un bain de glace une solution de 32 g (0,22 mole) de trans 4-hydroxy proline dans 200 ml d'eau que l'on agite vigoureusement. Par l'intermédiaire d'une ampoule à brome, on ajoute goutte à goutte 22,8 g (0,1 mole) le chlorure de 4,5-méthylène dioxy-2-nitro benzoyle obtenu dans la préparation 1 en solution dans 150 ml de l'acétone.Simultanément, une solution d'hydroxyde de sodium à 40% est également ajoutée goutte à goutte de façon à ce que le PH du mélange réactionnel demeure alcalin. On continue l'agitation après la fin de l'addition pendant 20 minutes à température ambiante. L'acétone est éliminée sous vide sans dépasser 40°C. La solution aqueuse est alors acidifiée puis abandonnée 24 heures à température ambiante. Les cristaux formés sont essorés, lavés à l'eau, séchés et recristallisés dans l'isopropanol.
Rendement : 75%
Point de fusion : 230°C
Caractéristiques spectrales :
v CO : 1730 et 1620 cm⁻¹
Résonance Magnétique Nucléaire ¹H :
O-CH₂-O δ = 6,25 ppm
=CH-C=O δ = 6,80 ppm
CH-CNO₂ δ = 7,65 ppm

| Composition centésimale : | | | |
|---|---|---|---|
| Calculée | C : 48,15 | H : 3,73 | N : 8,63 |
| Trouvée | C : 47,91 | H : 3,63 | N : 8,53 |

### EXEMPLE 1 : ACIDE 4-HYDROXY 1-(3,4-DIMETHOXY BENZOYL) PYRROLIDINE 2-CARBOXYLIQUE (TRANS)

En procédant comme dans la préparation 16 mais en utilisant le chlorure de 3,4-diméthoxy benzoyle obtenu dans la préparation 2 au lieu du chlorure de 4,5-méthylène dioxy 2-nitro benzoyle, on obtient le produit du titre.
Rendement : 60%
Point de fusion : 212°C
Caractéristiques spectrales :
v C=O à 1700 cm⁻¹
Résonance Magnétique Nucléaire :
OCH₃ δ = 3,80 ppm

| Composition centésimale : | | | |
|---|---|---|---|
| Calculée | C : 56,95 | H : 5,80 | N : 4,74 |
| Trouvée | C : 56,72 | H : 5,89 | N : 4,43 |

### EXEMPLE 2 : ACIDE 4-HYDROXY 1-(3,4 METHYLENE DIOXY BENZOYL) PYRROLIDINE 2-CARBOXYLIQUE (Forme trans)

En procédant comme dans l'exemple 1 et en remplaçant le chlorure de 3,4-diméthoxy benzoyle par le chlorure de 3,4-méthylène dioxy benzoyle obtenu dans la préparation 3, on obtient le produit du titre.
Recristallisation : isopropanol
Rendement : 70%
Point de fusion : 230°C
Caractéristiques spectrales :
Infrarouge :
v C=O à 1740 et 1630 cm⁻¹
Résonance Magnétique Nucléaire :
O-CH₂-O δ = 6,09 ppm
aromatiques : massif à 7 ppm

### EXEMPLE 3 : 4-HYDROXY 1-(3,4-DIMETHOXY BENZOYL) PYRROLIDINE 2-CARBOXAMIDE

A 3 g (0,01 mole) de l'acide 4-hydroxy 1-(3,4-diméthoxy benzoyl) pyrrolidine 2-carboxylique trans obtenu dans l'exemple 1 en suspension dans 200 ml d'acétone à 0°C on additionne, 1,1 équivalent de triéthylamine. Le mélange réactionnel est agité pendant 20 minutes puis on ajoute 1,1 équivalent de chloroformiate d'éthyle tout en maintenant la température entre 0°C et 5°C.
Après 20 minutes d'agitation, on filtre et on fait barboter dans la solution de l'ammoniac. Le précipité formé est séparé, on élimine sous vide l'acétone et le produit obtenu est recristallisé dans l'eau.
Rendement : 70%
Point de fusion : 240°C
Caractéristiques spectrales :
Infrarouge :
v C=O : 1675 et 1615 cm⁻¹
Résonance Magnétique Nucléaire :
O-CH₃ δ = 3,8 ppm

### EXEMPLE 4 : ACIDE 4-HYDROXY 1-(3,4,5-TRIMETHOXY BENZOYL)PYRROLIDINE 2-CARBOXYLIQUE

En procédant comme dans l'exemple 1, mais en utilisant le chlorure de 3,4,5-triméthoxy benzoyle obtenu dans la préparation 4 au lieu du chlorure de 3,4-diméthoxy benzoyle, on obtient le produit du titre.
Rendement : 50%
Point de fusion : 202°C
Caractéristiques spectrales :
Infrarouge :
v C=O : 1740 cm⁻¹
Résonance Magnétique Nucléaire :
O-CH₃ δ = 3,8 ppm
aromatiques : δ = 6-8 ppm

| Composition centésimale : | | | |
|---|---|---|---|
| Calculée | C : 55,38 | H : 5,88 | N : 4,30 |
| Trouvée | C : 55,34 | H : 5,74 | N : 4,31 |

### EXEMPLE 5 : 4-HYDROXY 1-[3,4-METHYLENEDIOXY BENZOYL]PYRROLIDINE 2-CARBOXAMIDE

### Stade A : ACIDE 1-(3,4 METHYLENEDIOXY BENZOYL) PYRROLIDINE 2-CARBOXYLIQUE

En procédant comme dans l'exemple 1, mais en utilisant la proline au lieu de la trans-4-hydroxy proline et le chlorure de 3,4-méthylène dioxy benzoyle obtenu à la préparation 3 au lieu du chlorure de 3,4-diméthoxy benzoyle, on obtient le produit du titre.
Rendement : 65%
Point de fusion : 164°C
Caractéristiques spectrales :
v C=O à 1730 et 1620 cm⁻¹
Résonance Magnétique Nucléaire :
CH-COOH δ = 4,4 ppm
O-CH₂-O δ = 6,07 ppm
aromatiques : massif à 7 ppm

| Composition centésimale : | | | |
|---|---|---|---|
| Calculée | C : 59,31 | H : 4,97 | N : 5,32 |
| Trouvée | C : 59,31 | H : 5,04 | N : 5,19 |

### Stade B : 4-HYDROXY 1-[3,4-METHYLENEDIOXY BENZOYL]PYRROLIDINE 2-CARBOXAMIDE

A 4,5 g (0,016 mole) de l'acide 4-hydroxy 1-[3,4-méthylènedioxy benzoyl]pyrrolidine 2-carboxylique obtenu au stade précédent sont mis en suspension dans 130 ml d'acétonitrile à 0°C, on additionne 1,1 équivalent de triéthylamine. Le mélange réactionnel est agité pendant 20 minutes puis on ajoute 1,1 équivalent de chloroformiate d'éthyle tout en maintenant la température entre 0 et 5°C.
Après 20 minutes d'agitation on fait barboter dans le mélange réactionnel de l'ammoniac. Le précipité formé est séparé. Le filtrat est éliminé sous vide, le précipité obtenu est recristallisé dans l'acétonitrile.
Rendement : 47%
Point de fusion : 176°C
Caractéristiques spectrales :
Infrarouge :
v C=O : 1670 et 1600 cm⁻¹
Résonance Magnétique Nucléaire :
O-CH₂-O δ = 6,08 ppm
aromatiques :7,01 ppm

### EXEMPLE 6 : ACIDE 1-[3,4-ETHYLENEDIOXY BENZOYL]4-HYDROXY PYRROLIDINE 2-CARBOXYLIQUE

En procédant comme dans l'exemple 7, mais en remplaçant le chlorure de 3,4-diméthoxy benzoyle par le chlorure de 3,4-éthylène dioxy benzoyle obtenu dans la préparation 6, on obtient le produit du titre.
Rendement : 70%
Point de fusion : 142°C
Caractéristiques spectrales :
Infrarouge :
v C=O : 1725 cm⁻¹
Résonance Magnétique Nucléaire : (DMSOD₆)
O-CH₂-CH₂-O δ = 4,25 ppm
aroamtiques :6,95 ppm

### EXEMPLE 7 : 4-HYDROXY 1-[3,4-METHYLENEDIOXY BENZOYL]PYRROLIDINE 2-CARBOXYLATE DE METHYLE

0,06 mole d' acide 4-hydroxy 1-[3,4-méthylènedioxy benzoyl]pyrrolidine 2-carboxylique obtenu dans l'exemple 2 dans un mélange de 150 ml de méthanol et de 5 ml d'acide sulfurique (d = 1,84) sont chauffés au reflux pendant 7 heures. Le méthanol est éliminé sous vide. Le résidu est repris par de l'acétate d'éthyle, la phase organique est lavée au moyen d'une solution saturée d'hydrogénocarbonate de sodium puis par de l'eau jusqu'à l'obtention d'un pH neutre. L'acétate d'éthyle est séché sur sulfate de magnésium et éliminé sous vide. Le dérivé obtenu est recristallisé dans un mélange éther/acétone.
Rendement : 72%
Point de fusion : 142°C
Caractéristiques spectrales :
Infrarouge :
v OH : 3430 cm⁻¹
v C=O : 1730 cm⁻¹
Résonance Magnétique Nucléaire : (DMSOD₆)
δ = 3,65 ppm, CH₃
δ = 6,09 ppm, O-CH₂-O
δ = 7,03 ppm, aromatiques

### EXEMPLE 8 : 4-HYDROXY 1-[3,4-DIMETHOXY BENZOYL]PYRROLIDINE 2-CARBOXYLATE DE ETHYLE

En procédant comme dans l'exemple 7, mais en remplaçant l'acide 4-hydroxy 1-[3,4-méthylène dioxy benzoyl]pyrrolidine 2-carboxylique par l'acide 1-[3,4-diméthoxy benzoyl]pyrrolidine 2-carboxylique (exemple 1), on obtient le produit du titre. (solvant de recristallisation : isopropopanol).
Rendement : 50%
Point de fusion : 174°C
Caractéristiques spectrales :
Infrarouge :
v OH : 3340 cm⁻¹
v CO : 1750 cm⁻¹
v CO : 1610 cm⁻¹
Résonance Magnétique Nucléaire : (DMSOD₆)
δ = 3,65 ppm, CH₃ (COOCH₃)
δ = 3,80 ppm, 2 OCH₃
δ = 7,04 ppm, aromatiques

### EXEMPLE 9 : 1-(3,4-ETHYLENE DIOXY BENZOYL) 4-HYDROXY PYRROLIDINE 2-CARBOXYLATE DE METHYLE

En procédant comme dans l'exemple 7, mais en remplaçant l'acide 4-hydroxy 1-(3,4-méthylène dioxy benzoyl) pyrrolidine 2-carboxylique par l'acide 1-(3,4-éthylène dioxy benzoyl) 4-hydroxy pyrrolidine 2-carboxylique, on obtient le produit du titre.
Solvant de recristallisation : isopropanol
Rendement : 60%
Point de fusion : 185°C
Caractéristiques spectrales :
Infrarouge :
v OH : 3440 cm⁻¹
v CO : 1750 cm⁻¹
v CO : 1615 cm⁻¹
Résonance Magnétique Nucléaire : (DMSOD₆)
δ = 3,65 ppm, CH₃
δ = 4,27 ppm, O-CH₂CH₂-O
δ = 6,97 ppm, aromatiques

### EXEMPLE 10 : 1-(3,4-ETHYLENE DIOXY BENZOYL) 4-HYDROXY PYRROLIDINE 2-CARBOXAMIDE

En procédant comme dans l'exemple 5, mais en remplaçant l'acide 4-hydroxy 1-(3,4-méthylènedioxy benzoyl) pyrrolidine 2-carboxylique par l'acide 1-(3,4-éthylène dioxy benzoyl) 4-hydroxy pyrrolidine 2-carboxylique, on obtient le produit du titre.
Solvant de recristallisation : eau
Rendement : 60%
Point de fusion : 170°C
Caractéristiques spectrales :
Infrarouge :
v CO : 1690 cm⁻¹
Résonance Magnétique Nucléaire : (DMSOD₆)
δ = 4,27 ppm, O-CH₂CH₂-O
δ = 6,96 ppm, aromatiques

### EXEMPLE 11 : 4-HYDROXY 1-(3,4-METHYLENE DIOXY BENZOYL)PYRROLIDINE 2-CARBOXYLATE D'ETHYLE

En procédant comme dans l'exemple 7, mais en remplaçant le méthanol par l'éthanol, on obtient le produit du titre.
Solvant de recristallisation : isopropanol
Rendement : 68 %
Point de fusion : 98°C
Caractéristiques spectrales :
Infrarouge :
v OH : 3420 cm⁻¹
v CO : 1740, 1600 cm⁻¹
Résonance Magnétique Nucléaire : (DMSOD₆)
δ = 1,20 ppm, CH₃
δ = 6,09 ppm, O-CH₂-O
δ = 7,02 ppm, aromatiques

### EXEMPLE 12 : 4-HYDROXY 1-(3,4-DIMETHOXY BENZOYL)PYRROLIDINE 2-CARBOXYLATE D'ETHYLE

En procédant comme dans l'exemple 7, mais en remplaçant l'acide 4-hydroxy 1-(3,4-méthylène dioxy benzoyl) pyrrolidine 2-carboxylique par l'acide 4-hydroxy 1-(3,4 diméthoxy benzoyl) pyrrolidine 2-carboxylique et le méthanol par l'éthanol, on obtient le produit du titre.
Solvant de recristallisation : acétate d'éthyle
Rendement : 60%
Point de fusion : 128°C
Caractéristiques spectrales :
Infrarouge :
v OH : 3340 cm⁻¹
v CO : 1740 cm⁻¹
v CO : 1610 cm⁻¹
Résonance Magnétique Nucléaire : (DMSOD₆)
δ = 1,20 ppm, CH₃ (COOCH₂CH₃)
δ = 3,80 ppm, 2 OCH₃
δ = 7,07 ppm, aromatiques

### EXEMPLE 13 : ACIDE 4-HXDROXY 1-(4-CHLORO BENZOYL)PYRROLIDINE 2-CARBOXYLIQUE

En procédant comme dans l'exemple 1, mais en remplaçant le chlorure de (3,4-diméthoxy) benzoyle par le chlorure de 4-chloro benzoyle obtenu dans la préparation 8, on obtient le produit du titre.

### EXEMPLE 14 : ACIDE 4-HYDROXY 1-P.TOLUOYL PYRROLIDINE-2 CARBOXYLIQUE

En procédant comme dans l'exemple 1, mais en remplaçant le chlorure de (3,4-diméthoxy) benzoyle par le chlorure de para toluoyle, on obtient le produit du titre.

### EXEMPLE 15 : ACIDE 4-HYDROXY 1-(4-TRIFLUOROMETHYL BENZOYL) PYRROLIDINE 2-CARBOXYLIQUE

En procédant comme dans l'exemple 1, mais en remplaçant le chlorure de (3,4-diméthoxy) benzoyle par le chlorure de para trifluorométhyle benzoyle obtenu dans la préparation 7, on obtient le produit du titre.

### EXEMPLE 16 : ACIDE 4-HYDROXY 1-(O-BENZYL BENZOYL) PYRROLIDINE 2-CARBOXYLIQUE

En procédant comme dans l'exemple 1, mais en remplaçant le chlorure de (3,4-diméthoxy) benzoyle par le chlorure de 2-benzyle benzoyle obtenu dans la préparation 9, on obtient le produit du titre.

### EXEMPLE 17 : ACIDE 4-HYDROXY 1-(4-BENZYLOXY BENZOYL) PYRROLIDINE 2-CARBOXYLIQUE

En procédant comme dans l'exemple 1, mais en remplaçant le chlorure de (3,4-diméthoxy) benzoyle par le chlorure de 4-benzyloxy benzoyle obtenu dans la préparation 10, on obtient le produit du titre.

### EXEMPLE 18 : ACIDE 4-HYDROXY 1 -(3,5 DITERT.BUTYL 4-HYDROXY BENZOYL) PYRROLIDINE 2-CARBOXYLIQUE

En procédant comme dans l'exemple 1, mais en remplaçant le chlorure de (3,4-diméthoxy) benzoyle par le chlorure de 3,5 ditert.butyl 4-hydroxy benzoyle obtenu dans la préparation 11, on obtient le produit du titre.

### EXEMPLE 19: ACIDE 4-HYDROXY 1-[6-(BENZO [1,4] DIOXINE)CARBONYL] PYRROLIDINE 2-CARBOXYLIQUE

En procédant comme dans l'exemple 1, mais en remplaçant le chlorure de 3,4-diméthoxy benzoyle par le chlorure de 6-(benzo [1,4] dioxine) carbonyle obtenu dans la préparation 12, on obtient le produit du titre.

### EXEMPLE 20 : ACIDE 4-HYDROXY 1-(2,3-METHYLENE DIOXY BENZOYL) PYRROLIDINE 2-CARBOXYLIQUE

En procédant comme dans l'exemple 1, mais en remplaçant le chlorure de 3,4-diméthoxy benzoyle par le chlorure de 2,3-méthylène dioxy benzoyle obtenu dans la préparation 13, on obtient le produit du titre.

### EXEMPLE 21 : ACIDE 4-HYDROXY 1-(2,3 ETHYLENE DIOXY BENZOYL) PYRROLIDINE 2-CARBOXYLIQUE

En procédant comme dans l'exemple 1, mais en remplaçant le chlorure de 3,4-diméthoxy benzoyle par le chlorure de 2,3-méthylène dioxy benzoyle obtenu dans la préparation 14, on obtient le produit du titre.

### EXEMPLE 22 : 4-HYDROXY 1-(3,4-METHYLENE DIOXY BENZOYL)PYRROLIDINE 2-N'METHYLCARBOXAMIDE

En procédant comme dans l'exemple 5, mais en remplaçant l'ammoniac par la méthylamine, on obtient le produit du titre.

### EXEMPLE 23 : [4-HYDROXY 1-(3,4-METHYLENE DIOXY BENZOYL)PYRROLIDINYL-2]MORPHOLINO CETONE

A 4,5 g (0,016 mole) d'acide 4-hydroxy 1-(3,4-méthylène dioxy benzoyl) pyrrolidine 2-carboxylique obtenu dans l'exemple 2 sont mis en suspension dans 110 ml d'acétonitrile à 0°C. On additionne 1,1 équivalent de triéthylamine et on agite le milieu réactionnel pendant 30 minutes, ajouter ensuite 1,1 équivalent de chloroformiate d'éthyle tout en maintenant la température entre 0 et 5°C. Après 30 minutes d'agitation, filtrer, ajouter au filtrat 1,1 équivalent de morpholine. Agiter à température ambiante 2 heures un équivalent. Le précipité est séparé, le filtrat est évaporé sous vide. Recristalliser le résidu.

### EXEMPLE 24 : [4-HYDROXY 1-(3,4-METHYLENE DIOXY BENZOYL)PYRROLIDINYL-2] [4-(2,3,4-TRIMETHOXY BENZYL)PIPERAZINYL-1]CETONE

En procédant comme dans l'exemple 23, mais en remplaçant la morpholine par la 1-(2,3,4-triméthoxy benzyl) pipérazine, on obtient le produit du titre.

### EXEMPLE 25 : ESTER METHYLIQUE DE L'ACIDE 4-HYDROXY 1-(4-METHYLAMINO BENZOYL) PYRROLIDINE-2 CARBOXYLIQUE

En procédant comme dans l'exemple 1, mais en remplaçant le chlorure de 3,4-diméthoxy benzoyle par le chlorure de 4-méthylamino benzoyle et la trans 4-hydroxy proline par l'ester méthylique de la trans 4-hydroxy proline, on obtient le produit du titre.

### EXEMPLE 26 : ACIDE 4-HYDROXY 1-(4-DIMETHYLAMINO BENZOYL) PYRROLIDINE-2 CARBOXYLIQUE

En procédant comme dans l'exemple 1, mais en remplaçant le chlorure de 3,4-diméthoxy benzoyle par le chlorure de 4-diméthylamino benzoyle, on obtient le produit du titre.

### EXEMPLE 27 : 4-HYDROXY 1-(3,4-METHYLENEDIOXY BENZOYL) PYRROLIDINE-2 N' CYCLOHEXYL CARBOXAMIDE

En procédant comme dans l'exemple 23, mais en remplaçant la morpholine par la cyclohexylamine, on obtient le produit du titre. Recristallisation dans l'acétate d'éthyle.
Rendement : 55 %
Point de fusion : 172° C
Caractéristiques spectrales :
Infrarouge :
Bandes OH et NH à 3280 cm⁻¹
Bandes C = O à 1660 et 1635 cm⁻¹
Résonance Magnétique Nucléaire ¹H (DMSOD₆) :
OH δ = 4,95 ppm
NH δ = 7,70 ppm

| Composition centésimale : | | | |
|---|---|---|---|
| Calculée | C : 63,31 | H : 6,71 | N : 7,77 |
| Trouvé | C : 63,34 | H : 6,85 | N : 7,60 |

### EXEMPLE 28 : 4-HYDROXY 1-(3,4-METHYLENEDIOXY BENZOYL) PYRROLIDINE-2 N' PHENYL CARBOXAMIDE

En procédant comme dans l'exemple 23, mais en remplaçant la morpholine par l'aniline, on obtient le produit du titre.

### EXEMPLE 29 : 4-HYDROXY 1-(3,4-METHYLENEDIOXY BENZOYL PYRROLIDINE-2 N' BENZYL CARBOXAMIDE

En procédant comme dans l'exemple 23, mais en remplaçant la morpholine par la benzylamine, on obtient le produit du titre.

### EXEMPLE 30 : 4-HYDROXY 1-(3,4-METHYLENEDIOXY BENZOYL) PYRROLIDINE-2 N' (3,4,5-TRIMETHOXY PHENYL) CARBOXAMIDE

En procédant comme dans l'exemple 23, mais en remplaçant la morpholine par la 3,4,5-triméthoxy aniline, on obtient le produit du titre.

### EXEMPLE 31 : 4-HYDROXY 1-(3,4-DIMETHOXY BENZOYL) PYRROLIDINE-2 N' CYCLOHEXYL CARBOXAMIDE

En procédant comme dans l'exemple 23, mais en remplaçant l'acide 4-hydroxy 1-(3,4-méthylène dioxy benzoyl) pyrrolidine-2 carboxylique par l'acide 4-hydroxy 1-(3,4-diméthoxy benzoyl) pyrrolidine-2 carboxylique obtenu dans l'exemple 4 et la morpholine par la cyclohexylamine, on obtient le produit du titre. Recristallisation dans un mélange acétate d'éthyle isopropanol
Rendement : 55 %
Point de fusion : 208° C
Caractéristiques spectrales :
Infrarouge :
Bande OH à 3430 cm⁻¹
Bande NH à 3340 cm⁻¹
Bandes CO à 1660 et 1610 cm⁻¹
Résonance Magnétique Nucléaire ¹H (DMSOD₆) :
OCH₃ δ = 3,80 ppm
OH δ = 5,00 ppm
NH δ = 7,45 ppm

| Composition centésimale : | | | |
|---|---|---|---|
| Calculée | C : 63,81 | H : 7,49 | N : 7,44 |
| Trouvé | C : 63,47 | H : 7,56 | N : 7,42 |

### EXEMPLE 32 : 4-HYDROXY 1-(3,4-DIMETHOXY BENZOYL) PYRROLIDINE-2 N' CYCLOOCTYL CARBOXAMIDE

En procédant comme dans l'exemple 31, mais en remplaçant la cyclohexylamine par la cyclooctylamine on obtient le produit du titre Recristallisation : acétate d'éthyle
Rendement : 50 %
Point de fusion : 178° C
Caractéristiques spectrales
Infrarouge :
Bande OH à 3440 cm⁻¹
Bande NH à 3340 cm⁻¹
Bandes CO à 1650 et 1610 cm⁻¹
Résonance Magnétique Nucléaire ¹H (DMSOD₆) :
CH₂ (cyclooctyle) δ = 1,5 ppm
OCH₃ δ = 3,8 ppm
OH δ = 4,95 ppm
NH δ = 7,80 ppm

| Composition centésimale : | | | |
|---|---|---|---|
| Calculée | C : 63,32 | H : 7,97 | N : 6,92 |
| Trouvé | C : 63,74 | H : 7,60 | N : 6,63 |

### EXEMPLE 33 : 4-HYDROXY 1-(3,4-DIMETHOXY BENZOYL) PYRROLIDINE-2 N' CYCLOPROPYL CARBOXAMIDE

En procédant comme dans l'exemple 31, mais en remplaçant la cyclohexylamine par la cyclopropylamine on obtient le produit du titre Recristallisation : acétate d'éthyle
Rendement : 42 %
Point de fusion : 208° C
Caractéristiques spectrales :
Infrarouge :
Bande OH à 3440 cm⁻¹
Bande NH à 3340 cm⁻¹
Bandes CO à 1650 et 1610 cm⁻¹
Résonance Magnétique Nucléaire ¹H (DMSOD₆) :
CH (cyclopropyl) δ = 2,60 ppm
OCH₃ δ = 3,8 ppm
OH δ = 4,95 ppm
NH δ = 8,00 ppm

| Composition centésimale : | | | |
|---|---|---|---|
| Calculée | C : 61,06 | H : 6,63 | N : 8,37 |
| Trouvé | C : 60,67 | H : 6,59 | N : 8,06 |

### EXEMPLE 34 : 4-HYDROXY 1-(3,4-METHYLENE DIOXY BENZOYL) PYRROLIDINE-2 N' CYCLOOCTYL CARBOXAMIDE

En procédant comme dans l'exemple 23, mais en remplaçant la morpholine par la cyclooctylamine, on obtient le produit du titre.
Recristallisation : acétate d'éthyle
Rendement : 50 %
Point de fusion : 188° C
Caractéristiques spectrales :
Infrarouge :
Bandes OH et NH à 3290 cm⁻¹
Bandes CO à 1670 et 1630 cm⁻¹
Résonance Magnétique Nucléaire ¹H (DMSOD₆)
OH δ = 4,95 ppm
NH δ = 7,75 ppm

| Composition centésimale : | | | |
|---|---|---|---|
| Calculée | C : 64,93 | H : 7,26 | N : 7,21 |
| Trouvé | C : 64,40 | H : 7,39 | N : 7,19 |

### EXEMPLE 35 : 4-HYDROXY 1-(3,4-METHYLENE DIOXY BENZOYL) PYRROLIDINE-2 N' CYCLOPROPYL CARBOXAMIDE

En procédant comme dans l'exemple 23, mais en remplaçant la morpholine par la cyclopropylamine, on obtient le produit du titre.
Recristallisation :_acétate d'éthyle
Rendement : 52 %
Point de fusion : 180° C
Caractéristiques spectrales :
Infrarouge :
Bandes OH à 3330 cm⁻¹
Bandes NH à 3280 cm⁻¹
Bandes CO à 1680 cm⁻¹
Résonance Magnétique Nucléaire ¹H (DMSOD₆) :
CH (cyclopropyl) δ = 2,60 ppm
OH δ = 5,00 ppm
NH δ = 8,00 ppm

| Composition centésimale : | | | |
|---|---|---|---|
| Calculée | C : 60,37 | H : 5,69 | N : 8,80 |
| Trouvé | C : 59,97 | H : 5,84 | N : 8,48 |

### EXEMPLE 36 : 4-HYDROXY 1-(3,4-METHYLENE DIOXY BENZOYL) PYRROLIDINE-2 N' CYCLOEHEXYL METHYL CARBOXAMIDE

En procédant comme dans l'exemple 23, mais en remplaçant la morpholine par la cyclohexylméthylamine, on obtient le produit du titre.
Recristallisation : acétate d'éthyle
Rendement : 46 %
Point de fusion : 178° C
Caractéristiques spectrales :
Infrarouge :
Bandes OH et NH à 3290 cm⁻¹
Bandes CO à 1670 et 1650 cm⁻¹
Résonance Magnétique Nucléaire ¹H (DMSOD₆)
CH₂ (NHCH₂ cyclohexyle) δ = 2,90 ppm
OH δ = 4,95 ppm
NH δ = 7,85 ppm

| Composition centésimale : | | | |
|---|---|---|---|
| Calculée | C : 64,15 | H : 6,99 | N : 7,48 |
| Trouvé | C : 64,39 | H : 6,97 | N : 7,48 |

### EXEMPLE 37 : 4-HYDROXY 1-(3,4-DIMETHOXY BENZOYL) PYRROLIDINE-2 N'(CYCLOHEXYL METHYL) CARBOXAMIDE

En procédant comme dans l'exemple 31, mais en remplaçant la cyclohexylamine par la cyclohexymethylamine, on obtient le produit du titre.
Rendement : 45 %
Point de fusion : 158° C
Caractéristiques spectrales :
Infrarouge :
Bande OH et NH à 3290 cm⁻¹
Bande CO à 1660 cm⁻¹
Résonance Magnétique Nucléaire ¹H (DMSOD₆) :
CH₂ (NH-CH₂ cyclohexyle) δ = 2,90 ppm
OCH₃ δ = 3,8 ppm
OH δ = 4,95 ppm
NH δ = 7,85 ppm

| Composition centésimale : | | | |
|---|---|---|---|
| Calculée | C : 64,59 | H : 7,74 | N : 7,17 |
| Trouvé | C : 64,15 | H : 7,69 | N : 6,98 |

### EXEMPLE 38 : 4-HYDROXY 1-(3,4,5-TRIMETHOXY BENZOYL) PYRROLIDINE-2 N' (CYCLOHEXYL METHYL) CARBOXAMIDE

En procédant comme dans l'exemple 36, mais en remplaçant l'acide 4-hydroxy 1-(3,4-méthylène dioxy benzoyl) pyrrolidine-2 carboxylique par l'acide 4-hydroxy 1-(3,4,5-triméthoxy benzoyl) pyrrolidine 2-carboxylique décrit dans l'exemple 4, on obtient le produit du titre.
Recristallisation : acétate d'éthyle
Rendement : 77 %
Point de fusion : 152° C
Caractéristiques spectrales :
Infrarouge :
Bandes OH à 3300 cm⁻¹
Bandes C=O à 1665 et 1630 cm⁻¹
Résonance Magnétique Nucléaire ¹H (DMSOD₆) :
CH₂ (NHCH₂ cyclohexyle) δ = 2,90 ppm
OCH₃ δ = 3,80 ppm
OH δ = 4,95 ppm
NH δ = 7,90 ppm

| Composition centésimale : | | | |
|---|---|---|---|
| Calculée | C : 62,83 | H : 7,67 | N : 6,66 |
| Trouvé | C : 63,21 | H : 7,79 | N : 6,42 |

### EXEMPLE 39 : 4-HYDROXY 1-(3,4-METHYLENE DIOXY BENZOYL) PYRROLIDINE-2 [CARBOXYLATE DE 3-(di N'N' DIMETHYL AMINO) PROPYLE] (OXALATE)

Dans 2 fourneaux de 125 ml contenant chacun 30 ml de propanol-1 on ajoute 1 équivalent (0,007 mole) de sodium. Après une heure d'agitation on additionne dans l'un d'eux 2 g (0,007 mole) de l'acide 4-hydroxy-1-(3,4 methylène dioxy benzoyl) pyrrolidine 2-carboxylique (exemple 2) et dans l'autre 1 équivalent de N-(3-chloropropyl) N,N-diméthyl amine (chlorhydrate).

L'agitation est poursuivie pendant 1 heure. Le mélange contenant le dérivé aminopropyle est versé goutte à goutte au premier mélange obtenu. Ce mélange réactionnel est ensuite chauffé au reflux pendant 4 heures. Le propanol-1 est ensuite éliminé sous pression réduite. L'huile résiduelle est reprise par 50 ml d'eau puis cette phase aqueuse est extraite 3 fois par 50 ml d'acétate d'éthyle. Les phases organiques sont rassemblées, séchées sur sulfate de magnésium et éliminées sous vide.

On obtient une huile à laquelle on ajoute 5 ml de propanol ; à la solution ainsi obtenue, on ajoute un équivalent d'acide oxalique. Le mélange réactionnel est chauffé au reflux pendant 30 minutes puis après refroidissement il est versé dans 200 ml d'éther éthylique, après agitation l'oxalate est filtré.
Recristallisation : acétonitrile
Rendement : 61 %
Point de fusion : 120° C
Caractéristiques spectrales :
Infrarouge :
Bande OH à 3360 cm⁻¹
Bandes C=O à 1730 et 1630 cm⁻¹
Résonance Magnétique Nucléaire ¹H (DMSOD₆) :
2CH₃ δ = 2,70 ppm
3CH₂ propyl δ = 1,95 ; 3, 00 et 4,15 ppm

| Composition centésimale : | | | |
|---|---|---|---|
| Calculée | C : 52,86 | H : 5,76 | N : 6,16 |
| Trouvée | C : 53,08 | H : 5,81 | N : 5,84 |

### EXEMPLE 40 : 4-HYDROXY (3,4-METHYLENE DIOXY BENZOYL) PYRROLIDINE-2 [CARBOXYLATE DE 2-(N'N' DIMETHYL AMINO) ETHYLE] OXALATE

En procédant comme dans l'exemple 39, mais en remplaçant la N-(3 chloropropyl) N,N diméthylamine (chlorhydrate) par la N-(2-chloroéthyl)N,N diméthylamine (chlorhydrate), on obtient le produit du titre.
Recristallisation : acétonitrile / éther éthylique
Rendement : 40 %
Point de fusion : 128° C
Caractéristiques spectrales :
Infrarouge :
Bandes OH : 3400 cm⁻¹
Bandes C=O : 1750, 1720 et 1630 cm⁻¹
Résonance Magnétique Nucléaire ¹H (DMSOD₆) :
2CH₃ δ = 2,80 ppm
2CH₂ (éthyle) δ = 3,30 et 4,40 ppm
OH δ = 5,05 ppm

| Composition centésimale : | | | |
|---|---|---|---|
| Calculée | C : 51,81 | H : 5,49 | N : 6,36 |
| Trouvé | C : 51,47 | H : 5,36 | N : 5,92 |

### EXEMPLE 41 : 4-HYDROXY 1-(3,4-METHYLENE DIOXY BENZOYL) PYRROLIDINE-2 (CARBOXYLATE DE 2-MORPHOLINO ETHYLE) (OXALATE)

En procédant comme dans l'exemple 39, mais en remplaçant la N-(3 chloropropyl) N,N diméthylamine (chlorhydrate) par la N-(2-chloroéthyl)morpholine (chlorhydrate), on obtient le produit du titre.
Recristallisation : acétonitrile
Rendement : 86 %
Point de fusion : 128° C
Caractéristiques spectrales :
Infrarouge :
Bandes OH à 3370 cm⁻¹
Bandes CO à 1745 et 1630 cm⁻¹
Résonance Magnétique Nucléaire ¹H (DMSOD₆) :
4CH₂ (morpholine) δ = 2,8 et 3,6 ppm

| Composition centésimale : | | | |
|---|---|---|---|
| Calculée | C : 52,88 | H : 5,43 | N : 5,80 |
| Trouvé | C : 52,42 | H : 5,44 | N : 6,25 |

### EXEMPLE 42 : 4-HYDROXY 1-(3,4-DIMETHOXYBENZOYL) PYRROLIDINE-2 (CARBOXYLATE DE 2-MORPHOLINO ETHYLE) (OXALATE)

En procédant comme dans l'exemple 41, mais en remplaçant l'acide 4-hydroxy 1-(3,4-méthylène dioxy benzoyl)pyrrolidine-2 carboxylique par l'acide 4-hydroxy 1-(3,4-diméthoxy benzoyl)pyrrolidine 2-carboxylique obtenu dans l'exemple 1, on obtient le produit du titre.
Recristallisation : acétonitrile
Rendement : 40 %
Point de fusion : 150°C
Caractéristiques spectrales :
Infrarouge :
Bande OH à 3460 cm⁻¹
Résonance Magnétique Nucléaire ¹H (DMSOD₆) :
4CH₂ (morpholine) δ = 2,7 et 3,6 ppm
OCH₃ δ = 3,80 ppm

### EXEMPLE 43 : 4-HYDROXY 1-(3,4-METHYLENE DIOXY BENZOYL) PYRROLIDINE-2 (CARBOXYLATE DE 2-PIPERIDINO ETHYLE) (OXALATE)

En procédant comme dans l'exemple 39, mais en remplaçant la N(3-chloropropyl)N,N diméthylamine chlorhydrate par la N-2 chloroéthyl pipéridine (chlorhydrate), on obtient le produit du titre.
Recristallisation : acétonitrile
Rendement : 80 %
Point de fusion : 128°C
Caractéristiques spectrales :
Infrarouge :
Bandes OH à 3460 cm⁻¹
Bandes CO à 1745 et 1630 cm⁻¹
Résonance Magnétique Nucléaire ¹H (DMSOD₆) :
4CH₂ (pipéridine) δ = 2,00 et 3,05 ppm

| Composition centésimale : | | | |
|---|---|---|---|
| Calculée | C : 54,99 | H : 5,87 | N : 5,83 |
| Trouvé | C : 54,91 | H : 5,90 | N : 6,24 |

### EXEMPLE 44 : 4-HYDROXY 1-(3,4-METHYLENE DIOXY BENZOYL) PYRROLIDINE-2 [CARBOXYLATE DE 3-(N,N DIETHYLAMINO)PROPYLE] (OXALATE)

En procédant comme dans l'exemple 39, mais en remplaçant la N(3-chloropropyl)N,N diméthylamine (chlorhydrate) par la N(3-chloropropyl)N,N diéthylamine (chlorhydrate), on obtient le produit du titre.
Recristallisation : éther éthylique
Rendement : 64 %
Point de fusion : 130°C
Caractéristiques spectrales :
Infrarouge :
Bandes CO à 1740 et 1625 cm⁻¹
Résonance Magnétique Nucléaire ¹H (DMSOD₆) :
2CH₃ (éthyle) δ = 0,9 ppm
2CH₂ (éthyle) δ = 2,40 ppm
OH δ = 5,10 ppm

### EXEMPLE 45 : 4-HYDROXY 1-(3,4-DIMETHOXY BENZOYL) PYRROLIDINE 2-N'N' DIETHYL CARBOXAMIDE

4 g (0,013 mole) de l'acide 4-hydroxy-1-(3,4-diméthoxy benzoyl) pyrrolidine 2-carboxylique (obtenu dans l'exemple 1) sont mis en suspension dans 100 ml d'acétonitrile à 0°C. On additionne 1,1 équivalent de triéthylamine. Après 20 minutes d'agitation on ajoute 1,1 équivalent de chloroformiate d'éthyle tout en maintenant la température entre 0 et 5°C. Le mélange réactionnel est agité pendant 20 minutes puis on additionne 1,1 équivalent de diéthylamine. Ce mélange est ensuite agité à température ambiante pendant 2 heures. L'acétonitrile est évaporé sous vide, l'huile résiduelle est reprise par 100 ml d'eau puis cette phase aqueuse est extraite par 3 fois 100 ml d'acétate d'éthyle. Ces phases organiques sont rassemblées, séchées et éliminées sous vide. L'huile obtenue cristallise après addition d'éther éthylique.
Recristalliser dans l'acétate d'éthyle
Rendement : 50 %
Point de fusion : 152°C
Caractéristiques spectrales :
Infrarouge :
Bande OH à 3440 cm⁻¹
Bande CO à 1645 et 1610 cm⁻¹
Résonance Magnétique Nucléaire ¹H (DMSOD₆) :
2(NCH₂CH₃) : 1,00 et 1,25 ppm
OCH₃ δ = 3,80 ppm
2(N-CH₂CH₃) : 3,18 et 3,50 ppm

| Composition centésimale : | | | |
|---|---|---|---|
| Calculée | C : 61,59 | H : 7,47 | N : 7,99 |
| Trouvé | C : 61,74 | H : 7,36 | N : 7,83 |

### EXEMPLE 46 : 1-(3,4-DIMETHOXY BENZOYL) PYRROLIDINE-2,4 CARBOLACTONE

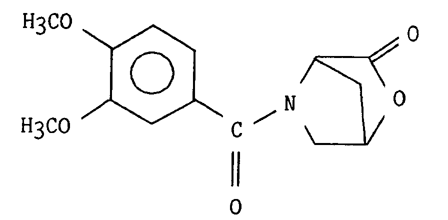

En utilisant le même procédé que celui décrit dans l'exemple précédent mais en remplaçant la diéthylamine par l'imidazole, on obtient le produit du titre.
Rendement : 32 %
Point de fusion : 132°C
Caractéristiques spectrales :
Infrarouge :
Bandes CO à 1800 et 1630 cm⁻¹
Résonance Magnétique Nucléaire ¹H (DMSOD₆) :
OCH₃ δ = 3,80 ppm

| Composition centésimale : | | | |
|---|---|---|---|
| Calculée | C : 60,64 | H : 5,45 | N : 5,05 |
| Trouvé | C : 60,46 | H : 5,48 | N : 4,92 |

### EXEMPLE 47 : 4-HYDROXY 1-(3,4-METHYLENE DIOXY BENZOYL) PYRROLIDINE-2-N,N' DIMETHYL CARBOXAMIDE

En procédant comme dans l'exemple 45, mais en remplaçant l'acide 4-hydroxy 1-(3,4-diméthoxy benzoyl)pyrrolidine 2-carboxylique par l'acide 4-hydroxy 1-(3,4-méthylene dioxy benzoyl)pyrrolidine 2-carboxylique (obtenu dans l'exemple 2) et la N,N diéthylamine par la N,N diméthylamine, on obtient le produit du titre.
Rendement : 50 %
Point de fusion : 170°C
Caractéristiques spectrales :
Infrarouge :
Bande OH à 3450 cm⁻¹
Bande CO à 1640 cm⁻¹
Résonance Magnétique Nucléaire ¹H (DMSOD₆) :
2CH₃ : 3,10 et 2,83 ppm
O-CH₂-O 6,08 ppm

### EXEMPLE 48 : 4-HYDROXY 1-(3,4,5-TRIMETHOXY BENZOYL) PYRROLIDINE-2-CARBOXAMIDE

En procédant comme dans l'exemple 5, mais en remplaçant l'acide 4-hydroxy 1-(3,4-méthylène dioxy benzoyl)pyrrolidine 2-carboxylique par l'acide 4-hydroxy 1-(3,4,5-triméthoxy benzoyl)pyrrolidine 2-carboxylique obtenu dans l'exemple 4, on obtient le produit du titre.
Rendement : 60 %
Point de fusion : 172°C
Caractéristiques spectrales :
Infrarouge :
Bande CO à 1665 et 1620 cm⁻¹
Résonance Magnétique Nucléaire ¹H (DMSOD₆) :
3OCH₃ 3,8 ppm
OH 4,96 ppm

| Composition centésimale : | | | |
|---|---|---|---|
| Calculée | C : 55,54 | H : 6,21 | N : 8,63 |
| Trouvé | C : 55,28 | H : 5,99 | N : 8,03 |

### EXEMPLE 49 : 4-HYDROXY 1-(3,4 ETHYLENE DIOXY BENZOYL) PYRROLIDINE-2-CARBOXYLATE D'ETHYLE

En procédant comme dans l'exemple 7, mais en remplaçant :
- l'acide 4-hydroxy 1-(3,4-méthylène dioxy benzoyl)pyrrolidine 2-carboxylique par l'acide 4-hydroxy 1-(3,4-éthylène dioxy benzoyl)pyrrolidine 2-carboxylique obtenu dans l'exemple 6, - le méthanol par l'éthanol,
on obtient le produit du titre.
Recristallisation : acétate d'éthyle
Rendement : 70 %
Point de fusion : 108°C
Caractéristiques spectrales :
Infrarouge :
Bandes OH : 3420 cm⁻¹
Bandes CO : 1740, 1610 cm⁻¹
Résonance Magnétique Nucléaire ¹H (DMSOD₆) :
COOCH₂CH₃ : 1,19 ppm
OCH₂CH₂O : 4,28 ppm
COOCH₂CH₃ : 4,13 ppm
OH : 5,12 ppm

| Composition centésimale : | | | |
|---|---|---|---|
| Calculée | C : 59,80 | H : 5,95 | N : 4,35 |
| Trouvé | C : 59,81 | H : 5,80 | N : 4,31 |

### EXEMPLE 50 : 4-HYDROXY 1-(3,4,5-TRIMETHOXY BENZOYL) PYRROLIDINE-2-CARBOXYLATE DE METHYLE

En procédant comme dans l'exemple 7, mais en remplaçant l'acide 4-hydroxy 1-(3,4-méthylène dioxy benzoyl)pyrrolidine 2-carboxylique par l'acide 4-hydroxy 1-(3,4,5-triméthoxy benzoyl)pyrrolidine 2-carboxylique obtenu dans l'exemple 4, on obtient le produit du titre.
Recristallisation : acétate d'éthyle
Rendement : 40 %
Point de fusion : 126°C
Caractéristiques spectrales :
Infrarouge :
Bande OH : 3485 cm⁻¹
Bande CO : 1730 cm⁻¹
Résonance Magnétique Nucléaire ¹H (DMSOD₆) :
COOCH₃ : 3,66 ppm
3OCH₃ : 3,80 ppm
OH : 5,10 ppm

| Composition centésimale : | | | |
|---|---|---|---|
| Calculée | C : 56,63 | H : 6,23 | N : 4,12 |
| Trouvé | C : 55,08 | H : 6,24 | N : 3,95 |

### EXEMPLE 51 : ACIDE 4-HYDROXY 1-(4-METHOXY BENZOYL) PYRROLIDINE-2-CARBOXYLIQUE

En procédant comme dans l'exemple 1, mais en remplaçant le chlorure de 4,5-méthylène dioxy 2-nitro benzoyle par le chlorure de 4-méthoxy benzoyle obtenu dans la préparation 15, on obtient le produit du titre.
Rendement : 50 %
Point de fusion : 192°C
Caractéristiques spectrales :
Infrarouge :
Bande OH : 3500, 2940 cm⁻¹
Bande CO : 1740 cm⁻¹
Résonance Magnétique Nucléaire ¹H (DMSOD₆) :
OCH₃ : 3,80 ppm
CHOH : 5,03 ppm
COOH : 12,71 ppm

| Composition centésimale : | | | |
|---|---|---|---|
| Calculée | C : 58,86 | H : 5,69 | N : 5,28 |
| Trouvé | C : 58,80 | H : 5,69 | N : 5,24 |

### EXEMPLE 52 : 4-HYDROXY 1-(4-METHOXY BENZOYL) PYRROLIDINE-2-CARBOXYLATE DE METHYLE

En procédant comme dans l'exemple 7, mais en remplaçant l'acide 4-hydroxy 1-(3,4-méthylène dioxy benzoyl) pyrrolidine 2-carboxylique par l'acide 4-hydroxy-1-(4-méthoxy benzoyl) pyrrolidine 2-carboxylique obtenu dans l'exemple 51, on obtient le produit du titre.
Recristallisation : éther éthylique
Rendement : 40 %
Point de fusion : 92°C
Caractéristiques spectrales :
Infrarouge :
Bande OH : 3480 cm⁻¹
Bande CO : 1740, 1605 cm⁻¹
Résonance Magnétique Nucléaire ¹H (DMSOD₆) :
COOCH₃ : 3,65 ppm
OH : 5,11 ppm

| Composition centésimale : | | | |
|---|---|---|---|
| Calculée | C : 60,20 | H : 6,13 | N : 5,01 |
| Trouvé | C : 60,10 | H : 5,98 | N : 4,96 |

### EXEMPLE 53 : 4-HYDROXY 1-(3,4,5-TRIMETHOXY BENZOYL) PYRROLIDINE-2-CARBOXYLATE D'ETHYLE

En procédant comme dans l'exemple 50, mais en remplaçant le méthanol par l'éthanol, on obtient le produit du titre.
Recristallisation : éther éthylique
Rendement : 70 %
Point de fusion : 78°C
Caractéristiques spectrales :
Infrarouge :
Bandes OH : 3480 cm⁻¹
Bandes CO : 1720 cm⁻¹
Résonance Magnétique Nucléaire ¹H (DMSO₆) :
COOCH₂CH₃ : 1,19 ppm
COOCH₂ - CH₃ : 4,11 ppm
OH : 5,11 ppm

### EXEMPLE 54 : 4-HYDROXY 1-(4-METHOXY BENZOYL) PYRROLIDINE-2-CARBOXYLATE D'ETHYLE

En procédant comme dans l'exemple 52, mais en remplaçant le méthanol par l'éthanol, on obtient le produit du titre.
Recristallisation : éther éthylique / éther de pétrole
Rendement : 50 %
Point de fusion : 64°C
Caractéristiques spectrales :
Infrarouge :
Bandes OH : 3360 cm⁻¹
Bandes CO : 1745, 1610 cm⁻¹
Résonance Magnétique Nucléaire ¹H (DMSOD₆) :
COOCH₂CH₃ : 1,19 ppm
OCH₃ : 3,80 ppm
COOCH₂CH₃ : 4,11 ppm
OH : 5,11 ppm

| Composition centésimale : | | | |
|---|---|---|---|
| Calculée | C : 61,42 | H : 6,42 | N : 4,77 |
| Trouvé | C : 61,08 | H : 6,35 | N : 4,76 |

### EXEMPLE 55 : [4-HYDROXY 1-(3,4-DIMETHOXY BENZOYL) PYRROLIDINE-2][4-(2,3,4-TRIMETHOXY BENZYL PIPERAZINYL-1] CETONE

En procédant comme dans l'exemple 24, mais en remplaçant l'acide 4-hydroxy 1-(3,4-méthylène dioxy benzoyl) pyrrolidine-2 carboxylique par l'acide 4-hydroxy 1-(3,4-diméthoxy benzoyl) pyrrolidine-2 carboxylique, on obtient le produit du titre.

### ETUDE PHARMACOLOGIQUE DES PRODUITS DE L'INVENTION

### EXEMPLE 56 : ETUDE DE LA TOXICITE AIGUE

La toxicité aigue a été déterminée après administration intrapéritonéale des produits de l'invention à des groupes de souris mâles de 25 g environ. Les animaux ont été observés à intervalles réguliers pendant les premières heures suivant l'administration et puis quotidiennement la semaine suivant l'administration.

Les dérivés de l'invention sont peu toxiques puisqu'aucun d'eux n'a une DL₅₀ (dose léthale 50, qui entraîne le décès de la moitié des animaux traités) inférieure à 500 mg.kg⁻¹.

### EXEMPLE 57 : AMNESIE INDUITE PAR LA SCOPOLAMINE

La scopolamine (1 mg.kg⁻¹ ip) est injectée 30 minutes et les produits à tester 60 minutes avant le début du test d'entraînement. L'appareillage utilisé comprend deux compartiments : l'un éclairé et l'autre sombre et posséde un plancher électrifié. La souris est introduite dans le compartiment éclairé, lorsqu'elle entre dans le compartiment sombre, elle reçoit une faible décharge électrique.

Après un entraînement 24 heures auparavant, le test consiste à mesurer le temps de latence entre deux entrées dans le compartiment sombre. Par rapport à un animal non traité, la scopolamine raccourcit ce temps à une dose de 0,03 mg.kg⁻¹.

Les dérivés de l'invention antagonisent l'amnésie induite par la scopolamine par augmentation de ce délai d'environ 75%.

### EXEMPLE 58 : COMPOSITION PHARMACEUTIQUE : COMPRIME

Comprimés dosés à 10 mg de Trans (L)-1-(3,4-diméthoxy benzoyl) 4-hydroxy pyrrolidine -2-N'N' diéthyl carboxamide.
Formule de préparation pour 1000 comprimés.

| | |
|---|---|
| Trans (L)-1-(3,4-diméthoxy benzoyl)4-hydroxy pyrrolidine 2-N',N'-diéthyl carboxamide | 10 g |
| Amidon de blé | 10 g |
| Amidon de maïs | 10 g |
| Lactose | 60 g |
| Stéarate de Magnesium | 2 g |
| Silice | 1 g |
| Hydroxy propyl cellulose | 2 g |

## Revendications (Revendications pour l'(les) Etat(s) contractant(s) suivant(s): AT, BE, CH, DE, DK, FR, GB, IT, LI, LU, NL, SE)

1. Composés de formule générale (I) : 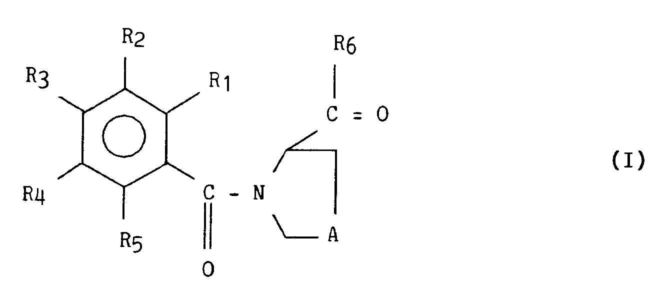 avec A représentant un groupement CO ou CHOH,
R₁, R₂, R₃, R₄, R₅ identiques ou différents représentant indépendamment l'un de l'autre un atome d'hydrogène, de chlore ou de fluor un groupement alkyle inférieur, cycloalkyle de 3 à 8 atomes de carbone, un groupement hydroxy, un groupement alkoxy inférieur, un groupement alkyle inférieur amino, un groupement dialkyl inférieur amino, un groupement phénylalkyle inférieur, un groupement phénylalkyle inférieur oxy, un groupement alkyle inférieur substitué par un ou plusieurs atomes d'halogène, ou (et) encore deux groupements adjacents parmi R₁, R₂, R₃, R₄, R₅ c'est à dire R₁ et R₂, ou R₂ et R₃ ou R₃ et R₄ ou R₄ et R₅ forment ensemble un pont -O-CH₂-O-,-O-CH₂-CH₂-O-, ou -O-CH=CH-O- étant entendu que :
. R₁, R₂, R₃, R₄, R₅ ne peuvent représenter simultanément un atome d'hydrogène,
. A ne peut représenter un groupement C=O que lorsque R₁ et R₂ ou R₂ et R₃ ou R₃ et R₄ ou R₄ et R₅ forment ensemble un pont -O-CH₂-O- ou -O-CH₂-CH₂-O- ou -O-CH=CH-O-, R₆ représente un groupement hydroxy où un groupement alkoxy inférieur, un groupement amino ou NR₇R₈ ou -O-B-NR₇R₈, avec B alkyle inférieur et R₇, R₈ identiques ou différents représentant un atome d'hydrogène, un groupement alkyle inférieur, ou cycloalkyle ou cycloalkylalkyle inférieur, phényle, phénylalkyle inférieur, phényle substitué ou phénylalkyle inférieur substitué, ou R₇ et R₈ forment avec l'azote qui les porte un système hétérocyclique mono, ou bicyclique, chaque cycle comprenant de cinq à six sommets et intégrant éventuellement dans son squelette de un à deux hétéroatomes choisi parmi azote, oxygène ou soufre et éventuellement substitué par un groupement alkyle inférieur ou phényle ou phényle alkyle inférieur, ou phénylalkyle inférieur substitué ou phényle substitué, le terme substitué affectant les termes phényle et phényle alkyle inférieur signifiant que le noyau aromatique des ces groupes peut être substitué par un ou plusieurs groupements alkyle inférieur, alkoxy inférieur, ou trifluorométhyle ou bien encore R₆ forme avec A un système lactonique ce qui confère aux dérivés de formule (I) la formule particulière (I') : 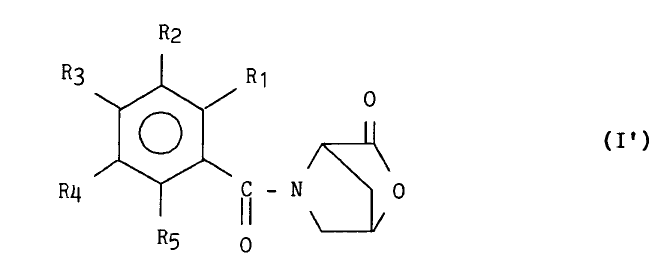 où R₁, R₂, R₃, R₄, R₅ ont la même définition que précédemment,
aux conditions que :
. si R₆ représente un groupement hydroxy et A représente un groupement CHOH ou R₆ et A forment ensemble un groupement lactonique interne (dérivés de formule (I')) on ne peut avoir simultanément R₁, R₄ et R₅ représentant chacun simultanément un atome d'hydrogène et R₂ et R₃ représentant chacun un atome de chlore, et symétriquement, R₅, R₁ et R₂ représentant un atome d'hydrogène et R₃ et R₄ représentant chacun un atome de chlore,
. si R₆ et A forment ensemble un groupement lactonique interne (dérives de formule (I')) au moins deux groupements parmi R₁, R₂, R₃, R₄ et R₅ sont différents de l'atome d'hydrogène,
. R₁ ou R₅ ne peuvent représenter un groupement acétyloxy si simultanément R₂, R₃, R₄, R₆ et l'autre groupement parmi R₁ et R₅ représente un atome d'hydrogène.
. étant entendu que par groupement cycloalkyle on entend des groupements de 3 à 8 atomes de carbone et par groupement alkyle inférieur on entend des groupements linéaire ou ramifié de 1 à 6 atomes de carbone,
ainsi que le cas échéant leurs isomères, épimères, diastéréoisomères et leurs sels d'addition à un acide ou une base pharmaceutiquement acceptable.

2. Composés de formule (I) selon la revendication 1 pour lesquels A représente un groupement CHOH, leurs isomères Trans (L) ou (2S,4R), trans (-D) ou (2R,4S), cis-(L) ou (2S,4S) ou cis (D)-(2R,4R) ainsi que le cas échéant, leurs sels d'addition à un acide ou une base pharmaceutiquement acceptable.

3. Composés de formule (I) selon la revendication 1, pour lesquels A représente un groupement CHOH ayant la configuration Trans (L) ou (2S,4R) et le cas échéant leurs sels d'addition à un acide ou une base pharmaceutiquement acceptable.

4. Composé de formule (I) selon la revendication 1, caractérisé en ce que R₂, R₃, R₄ identiques ou différents représentent un groupement alkoxy inférieur ou un atome d'hydrogène ou un groupement hydroxy tandis que R₁ et R₅ représentent chacun un groupement hydrogène, leurs isomères ainsi que leurs sels d'addition à un acide ou une base pharmaceutiquement acceptable.

5. Composé selon la revendication 1 pour lesquels R₆ représente un groupement hydroxyle ou alkoxy inférieur ou amino ou N,N dialkyle inférieur amino ou 4-(2,3,4-triméthoxy benzyl) pipérazinyl-1, leurs isomères ainsi que le cas échéant leurs sels d'addition à un acide ou une base pharmaceutiquement acceptable.

6. Composé selon la revendication 1 qui est le trans (L) 1-(3,4-diméthoxy benzoyl) 4-hydroxy pyrrolidine-2 N',N' diéthyl carboxamide, l'acide carboxylique correspondant, leurs isomères Trans (D), Cis (L), Cis (D) ainsi que ses sels d'addition à une base pharmaceutiquement acceptable..

7. Composé selon la revendication 1 qui est l'acide Trans (L) 1-(3,4,5-triméthoxy benzoyl) 4-hydroxy pyrrolidine-2-carboxylique, ses isomères Trans (D), Cis (L), Cis (D) ainsi que ses sels d'addition à une base pharmaceutiquement acceptable.

8. Composé selon la revendication 1 qui est le Trans (L) 1-(3,4-diméthoxy benzoyl) 4-hydroxy pyrrolidine-2 N'(cyclohexyl méthyl)carboxamide, ses isomères Trans (D), Cis (L), Cis (D).

9. Composé selon la revendication 1 qui est le Trans (L) 1-(3,4-diméthoxy benzoyl) 4-hydroxy pyrrolidine-2 N' cyclopropyl carboxamide, ses isomères Trans (D), Cis (L), Cis (D).

10. Composé selon la revendication 1 qui est le Trans (L) 1-(3,4-méthylène dioxy benzoyl) 4-hydroxy pyrrolidine-2 N'(cyclohexyl méthyl) carboxamide, ses isomères Trans (D), Cis (L), Cis (D).

11. Composé selon la revendication 1 qui est le Trans (L) 1-(3,4 méthylène dioxy benzoyl) 4-hydroxy pyrrolidine-2 carboxylate de 3-(N'N' diméthyl amino) propyle, ses isomères Trans (D), Cis (L), Cis (D) ainsi que ses sels d'addition à un acide pharmaceutiquement acceptable.

12. Composé selon la revendication 1 qui est le Trans (L)-1-(3,4-méthylène dioxy benzoyl) 4-hydroxy pyrrolidine-2 carboxylate de 2-morpholino éthyle, ses isomères Trans (D), Cis (L), Cis (D) ainsi que ses sels d'addition à un acide pharmaceutiquement acceptable.

13. Composé selon la revendication 1 qui est le Trans (L) 1-(3,4-diméthoxy benzoyl) 4-hydroxy pyrrolidine-2- carboxylate de 2-morpholino éthyle, ses isomères Trans (D), Cis (L), Cis (D) ainsi que ses sels d'addition à un acide pharmaceutiquement acceptable.

14. Composé selon la revendication 1 qui est la Trans (L)[4-hydroxy 1-(3,4-méthylène dioxy benzoyl) pyrrolidinyl-2][4-(2,3,4-triméthoxy benzyl) pipérazinyl-1]cétone, ses isomères Trans (D), cis (L), cis (D) ainsi que ses d'addition à un acide pharmaceutiquement acceptable.

15. Composé selon la revendication 1 qui est la Trans (L) [4-hydroxy 1-(3,4-diméthoxy benzoyl) pyrrolidinyl-2][4-(2,3,4-triméthoxy benzyl) pipérazinyl-1]cétone, ses isomères Trans (D), cis (L), (cis) D ainsi que ses d'addition à un acide pharmaceutiquement acceptable.

16. Composés selon la revendication 1 pour lesquels R₆ représente un groupement alkoxy inférieur ainsi que leurs isomères.

17. Composé selon la revendication 1 qui est le Trans (L) 1-(3,4,5-triméthoxy benzoyl) 4-hydroxy pyrrolidine-2 N'cyclohexyl méthyl carboxamide, ses isomères Trans (D), Cis (L), Cis (D).

18. Composé selon la revendication 1 qui est le Trans (L) 1-(3,4-diméthoxy benzoyl)4-hydroxy pyrrolidine-2N' cyclooctyl carboxamide, ses isomères Trans (D), Cis (L), Cis (D).

19. Composé selon la revendication 1 qui est le Trans (L) 1-(3,4-diméthoxy benzoyl) 4-hydroxy pyrrolidine-2 carboxamide, ses isomères Trans (D), Cis (L), Cis (D).

20. Composé selon la revendication 1, qui est le Trans (L) 1-(3,4,5-triméthoxy benzoyl) 4-hydroxy pyrrolidine-2 carboxylate d'éthyle, ses isomères Trans (D), Cis (L), Cis (D).

21. Procédé d'obtention des composés de formule générale (I) selon la revendication 1 caractérisé en ce que l'on utilise comme matière première un dérivé de formule (II) :
dans laquelle R₁, R₂, R₃, R₄ et R₅ ont la même définition que dans la formule (I) et X représente un atome d'halogène,
que l'on traite par le dérivé de formule (III) :
dans laquelle R₆ a la même définition que dans la formule (I), 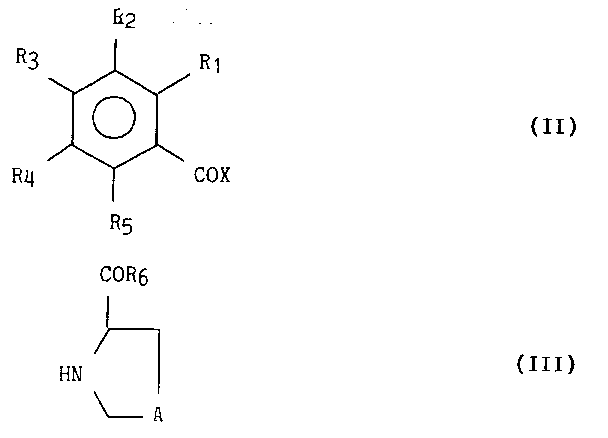 pour conduire à un dérivé de formule (I) : 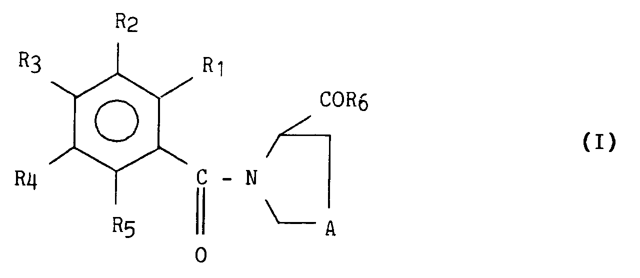 que l'on sépare le cas échéant en ses isomères et que l'on salifie, si on le désire, par un acide ou le cas échéant une base pharmaceutiquement acceptable,
dérivé de formule (I) qui, lorsque R₆ représente un groupement OH peut être traité le cas échéant, après éventuelle activation de la fonction acide carboxylique,
- ou bien par une amine de formule NR₇R₈ ou un dérivé de formule X-B-NR₇R₈ ou X représente un atome d'halogène et B, R₇ et R₈ ont la même signification que dans la formule (I) ou par un alcool aliphatique inférieur, R'6OH où R'₆ représente un groupement alkyle inférieur pour obtenir un composé de formule (I) pour lesquels R₆ représente respectivement un groupement NR₇R₈ ou un groupement alkyle inférieur,
- ou bien par l'imidazole pour obtenir un dérivé de formule (I'), dérivé de formule (I) ou (I') que l'on purifie que l'on purifie si on le désire, que l'on sépare le cas échéant en ses isomères et que l'on salifie si on le désire, par un acide ou une base pharmaceutiquement acceptable.

22. Procédé de préparation de dérivés de formule (I) pour lesquels A représente un groupement C=O caractérisé en ce que l'on traite un dérivé de formule (I/B) : 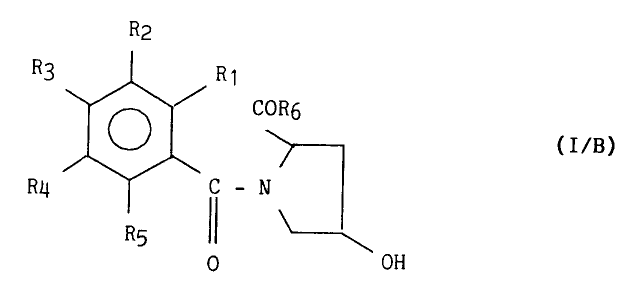 cas particulier des dérivés de formule (I) pour lesquels R₁, R₂, R₃, R₄, R₅ et R₆ ont la même définition que dans la formule (I) et A représente un groupement CHOH,
par un agent oxydant pour obtenir un dérivé de formule (I/C) : 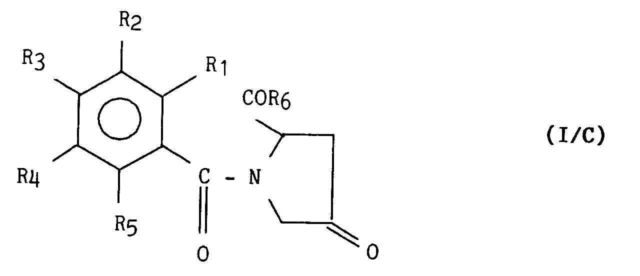 dans laquelle R₁, R₂, R₃, R₄, R₅ et R₆ ont la même définition que dans la formule (I),
que l'on purifie et que l'on salifie si on le désire, le cas échéant par un acide ou une base pharmaceutiquement acceptable,
et que l'on peut traiter, lorsque R₆ représente un groupement hydroxyle, en fonction du dérivé de formule (I) que l'on souhaite obtenir ou bien par une amine de formule NR₇R₈ où un dérivé X-B-NR₇R₈ où B, R₇ et R₈ ont la même signification que dans la formule (I) et X représente un atome d'halogène ou par un alcool aliphatique inférieur, R'₆OH avec R'₆ un groupement alkyle inférieur pour obtenir un composé de formule (I) pour lesquels R₆ représente respectivement un groupement NR₇R₈ ou un groupement alkyle inférieur,
que l'on purifie si nécessaire et dont on sépare le cas échéant les isomères et que l'on salifie, si on le désire et le cas échéant par un acide ou une base pharmaceutiquement acceptable.

23. Composition pharmaceutique contenant comme principe actif au moins un composé selon l'une des revendication 1 à 20 en combinaison avec un ou plusieurs excipients ou véhicules inertes non toxiques, pharmaceutiquement acceptables.

24. Composition pharmaceutique selon la revendication 23 contenant au moins un principe actif selon l'une des revendications 1 à 20 utilisables dans le traitement des maladies résultant de troubles hypoxiques ischémiques ou oxyprives, les syndromes du déficit intellectuel, la pathologie du sujet âgé, les troubles de l'attention, les infarctus cérébraux constitués et les vertiges d'origine centrale.

## Revendications (Revendications pour l'(les) Etat(s) contractant(s) suivant(s): ES, GR)

1. Procédé de préparation de composés de formule générale (I) : 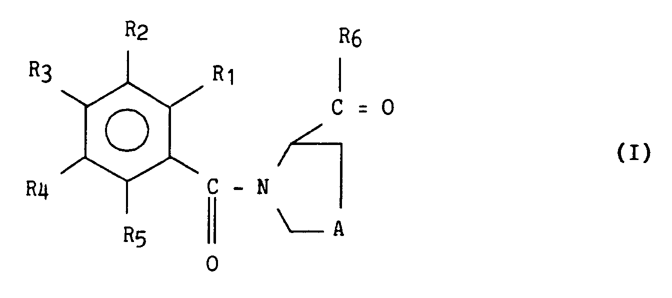 avec A représentant un groupement CO ou CHOH,
R₁, R₂, R₃, R₄, R₅ identiques ou différents représentant indépendamment l'un de l'autre un atome d'hydrogène, de chlore ou de fluor, un groupement alkyle inférieur, cycloalkyle de 3 à 8 atomes de carbone, un groupement hydroxy, un groupement alkoxy inférieur, un groupement alkyle inférieur amino, un groupement dialkyl inférieur amino, un groupement phénylalkyle inférieur, un groupement phénylalkyle inférieur oxy, un groupement alkyle inférieur substitué par un ou plusieurs atomes d'halogène, ou (et) encore deux groupements adjacents parmi R₁, R₂, R₃, R₄, R₅ c'est à dire R₁ et R₂, ou R₂ et R₃ ou R₃ et R₄ ou R₄ et R₅ forment ensemble un pont -O-CH₂-O,-O-CH₂-CH₂-O-, ou -O-CH=CH-O- étant entendu que :
. R₁, R₂, R₃, R₄, R₅ ne peuvent représenter simultanément un atome d'hydrogène,
. A ne peut représenter un groupement C=O que lorsque R₁ et R₂ ou R₂ et R₃ ou R₃ et R₄ ou R₄ et R₅ forment ensemble un pont -O-CH₂-O- ou -O-CH₂-CH₂-O- ou -O-CH=CH-O-,
R₆ représente un groupement hydroxy ou un groupement alkoxy inférieur, un groupement amino ou NR₇R₈ ou -O-B-NR₇R₈, avec B alkyle inférieur et R₇, R₈ identiques ou différents représentant un atome d'hydrogène, un groupement alkyle inférieur, ou cycloalkyle ou cycloalkylalkyle inférieur, phényle, phénylalkyle inférieur, phényle substitué ou phénylalkyle inférieur substitué, ou R₇ et R₈ forment avec l'azote qui les porte un système hétérocyclique mono, ou bicyclique, chaque cycle comprenant de cinq à six sommets et intégrant éventuellement dans son squelette de un a deux hétéroatomes choisi parmi azote, oxygène ou soufre et éventuellement substitué par un groupement alkyle inférieur ou phényle ou phényle alkyle inférieur, ou phénylalkyle inférieur substitué ou phényle substitué, le terme substitué affectant les termes phényle et phényle alkyle inférieur signifiant que le noyau aromatique des ces groupes peut être substitué par un ou plusieurs groupements alkyle inférieur, alkoxy inférieur, ou trifluorométhyle ou bien encore R₆ forme avec A un système lactonique ce qui confère aux dérivés de formule (I) la formule particulière (I') : 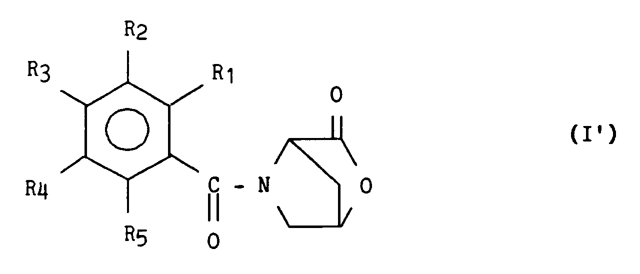 où R₁, R₂, R₃, R₄, R₅ ont la même définition que précédemment,
aux conditions que :
. si R₆ représente un groupement hydroxy et A représente un groupement CHOH ou R₆ et A forment ensemble un groupement lactonique interne (dérivés de formule (I')) on ne peut avoir simultanément R₁, R₄ et R₅ représentant chacun simultanément un atome d'hydrogène et R₂ et R₃ représentant chacun un atome de chlore, et symétriquement, R₅, R₁ et R₂ représentant un atome d'hydrogène et R₃ et R₄ représentant chacun un atome de chlore,
. si R₆ et A forment ensemble un groupement lactonique interne (dérivés de formule (I')) au moins deux groupements parmi R₁, R₂, R₃, R₄ et R₅ sont différents de l'atome d'hydrogène,
. R₁ ou R₅ ne peuvent représenter un groupement acétyloxy si simultanément R₂, R₃, R₄, R₆ et l'autre groupement parmi R₁ et R₅ représente un atome d'hydrogène.
. étant entendu que par groupement cycloalkyle on entend des groupements de 3 à 8 atomes de carbone et par groupement alkyle inférieur on entend des groupements linéaire ou ramifié de 1 à 6 atomes de carbone,
ainsi que le cas échéant leurs isomères, épimères, diastéréoisomères et leurs sels d'addition à un acide ou une base pharmaceutiquement acceptable,
caractérisé en ce que l'on utilise comme matière première un dérivé de formule (II) : 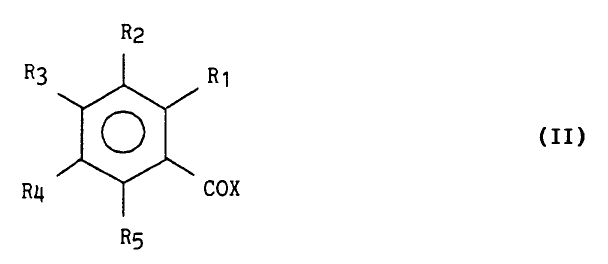 dans laquelle R₁, R₂, R₃, R₄ et R₅ ont la même définition que dans la formule (I) et X représente un atome d'halogène,
que l'on traite par le dérivé de formule (III) : 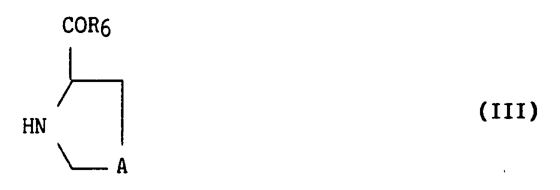 dans laquelle R₆ a la même définition que dans la formule (I),
pour conduire à un dérivé de formule (I) : 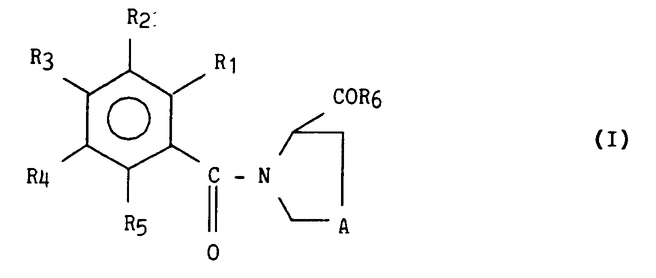 que l'on sépare le cas échéant en ses isomères et que l'on salifie, si on le désire, par un acide ou le cas échéant une base pharmaceutiquement acceptable,
dérivé de formule (I) qui, lorsque R₆ représente un groupement OH peut être traité le cas échéant, après éventuelle activation de la fonction acide carboxylique,
- ou bien par une amine de formule NR₇R₈ ou un dérivé de formule X-B-NR₇R₈ ou X représente un atome d'halogène et B, R₇ et R₈ ont la même signification que dans la formule (I) ou par un alcool aliphatique inférieur, R'6OH où R'₆ représente un groupement alkyle inférieur pour obtenir un composé de formule (I) pour lesquels R₆ représente respectivement un groupement NR₇R₈ ou un groupement alkyle inférieur,
- ou bien par l'imidazole pour obtenir un dérivé de formule (I'),
dérivé de formule (I) ou (I') que l'on purifie si on le désire, que l'on sépare le cas échéant en ses isomères et que l'on salifie si on le désire, par un acide ou une base pharmaceutiquement acceptable.

2. Procédé de préparation de dérivés de formule (I) pour lesquels A représente un groupement C=O caractérisé en ce que l'on traite un dérivé de formule (I/B) : 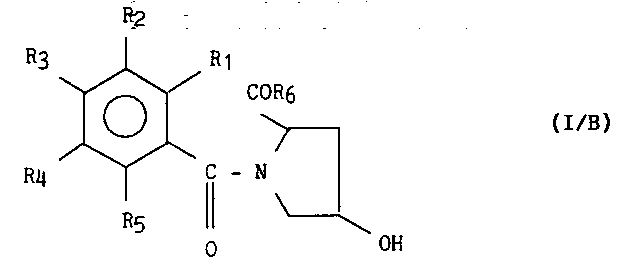 cas particulier des dérivés de formule (I) pour lesquels R₁, R₂, R₃, R₄, R₅ et R₆ ont la même définition que dans la formule (I) et A représente un groupement CHOH,
par un agent oxydant pour obtenir un dérivé de formule (I/C) : 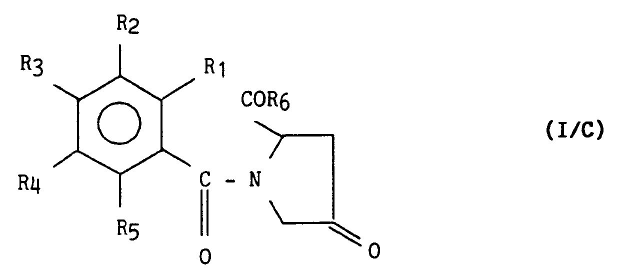 dans laquelle R₁, R₂, R₃, R₄, R₅ et R₆ ont la même définition que dans la formule (I),
que l'on purifie et que l'on salifie si on le désire, le cas échéant par un acide ou une base pharmaceutiquement acceptable,
et que l'on peut traiter, lorsque R₆ représente un groupement hydroxyle, en fonction du dérivé de formule (I) que l'on souhaite obtenir ou bien par une amine de formule NR₇R₈ où un dérivé X-B-NR₇R₈ où B, R₇ et R₈ ont la même signification que dans la formule (I) et X représente un atome d'halogène ou par un alcool aliphatique inférieur, R'₆OH avec R'₆ un groupement alkyle inférieur pour obtenir un composé de formule (I) pour lesquels R₆ représente respectivement un groupement NR₇R₈ ou un groupement alkyle inférieur,
que l'on purifie si nécessaire et dont on sépare le cas échéant les isomères et que l'on salifie, si on le désire et le cas échéant par un acide ou une base pharmaceutiquement acceptable.

3. Procédé de préparation selon la revendication 1 de composés de formule (I) pour lesquels A représente un groupement CHOH, leurs isomères trans (L) ou (2S, 4R), trans (-D) ou (2R,4S), cis-(L) ou (2S, 4S) ou cis D-(2R, 4R) ainsi que le cas échéant, leurs sels d'addition à un acide ou une base pharmaceutiquement acceptable.

4. Procédé de préparation selon la revendication 1 de composés de formule (I) pour lesquels A représente un groupement CHOH ayant la configuration TRANS-(L) ou (2S,4R) et le cas échéant leurs sels d'addition à un acide ou une base pharmaceutiquement acceptable.

5. Procédé de préparation selon la revendication 1 de composés de formule (I) selon la revendication 1, caractérisé en ce que R₂, R₃, R₄ identiques ou différents représentent un groupement alkoxy inférieur ou un atome d'hydrogène ou un groupement hydroxy tandis que R₁ et R₅ représentent chacun un groupement hydrogène, leurs isomères ainsi que leurs sels d'addition à un acide ou une base pharmaceutiquement acceptable.

6. Procédé de préparation selon la revendication 1 du composé de formule (I) pour lesquels R₆ représente un groupement hydroxyle ou alkoxy inférieur ou amino ou N,N dialkyle inférieur amino ou 4-(2,3,4-triméthoxy benzyl) pipérazinyl-1, leurs isomères ainsi que le cas échéant leurs sels d'addition à un acide ou une base pharmaceutiquement acceptable.

7. Procédé de préparation selon la revendication 1 du composé de formule (I) qui est le Trans (L) 1-(3,4-diméthoxy benzoyl) 4-hydroxy pyrrolidine-2 N',N' diéthyl carboxamide, l'acide carboxylique correspondant, leurs isomères Trans (D), Cis (L), Cis (D) ainsi que ses sels d'addition à une base pharmaceutiquement acceptable.

8. Procédé de préparation selon la revendication 1 du composé de formule (I) qui est l'acide Trans (L) 1-(3,4,5-triméthoxy benzoyl) 4-hydroxy pyrrolidine-2-carboxylique, ses isomères Trans (D), Cis (L), Cis (D) ainsi que ses sels d'addition à une base pharmaceutiquement acceptable.

9. Procédé de préparation selon la revendication 1 du composé de formule (I) qui est le Trans (L) 1-(3,4-diméthoxy benzoyl) 4-hydroxy pyrrolidine-2 N'(cyclohexyl méthyl)carboxamide, ses isomères Trans (D), Cis (L), Cis (D).

10. Procédé de préparation selon la revendication 1 du composé de formule (I) qui est le Trans (L) 1-(3,4-diméthoxy benzoyl) 4-hydroxy pyrrolidine-2 N' cyclopropyl carboxamide, ses isomères Trans (D), Cis (L), Cis (D).

11. Procédé de préparation selon la revendication 1 du composé de formule (I) qui est le Trans (L) 1-(3,4-méthylène dioxy benzoyl) 4-hydroxy pyrrolidine-2 N'(cyclohexyl méthyl) carboxamide, ses isomères Trans (D), Cis (L), Cis (D).

12. Procédé de préparation selon la revendication 1 du composé de formule (I) qui est le Trans (L) 1-(3,4 méthylène dioxy benzoyl) 4-hydroxy pyrrolidine-2 carboxylate de 3-(N'N' diméthyl amino) propyle, ses isomères Trans D, Cis L, Cis D ains que ses sels d'addition à un acide pharmaceutiquement acceptable.

13. Procédé de préparation selon la revendication 1 du composé de formule (I) qui est le Trans (L) 1-(3,4-méthylène dioxy benzoyl) 4-hydroxy pyrrolidine-2 carboxylate de 2-morpholino éthyle, ses isomères Trans (D), Cis (L), Cis (D) ainsi que ses sels d'addition à un acide pharmaceutiquement acceptable.

14. Procédé de préparation selon la revendication 1 du composé de formule (I) qui est le Trans (L) 1-(3,4-diméthoxy benzoyl) 4-hydroxy pyrrolidine-2- carboxylate de 2-morpholino éthyle, ses isomères Trans (D), Cis (L), Cis (D) ainsi que ses sels d'addition à un acide pharmaceutiquement acceptable.

15. Procédé de préparation selon la revendication 1 du composé de formule (I) qui est la Trans (L)[4-hydroxy 1-(3,4-méthylène dioxy benzoyl) pyrrolidinyl-2][4-(2,3,4-triméthoxy benzyl) pipérazinyl-1]cétone, ses isomères Trans (D), cis (L), cis (D) ainsi que ses d'addition à un acide pharmaceutiquement acceptable.

16. Procédé de préparation selon la revendication 1 du composé de formule (I) qui est la Trans (L) [4-hydroxy 1-(3,4-diméthoxy benzoyl) pyrrolidinyl-2][4-(2,3,4-triméthoxy benzyl) pipérazinyl-1]cétone, ses isomères Trans (D), cis (L), cis (D) ainsi que ses d'addition à un acide pharmaceutiquement acceptable.

17. Procédé de préparation selon la revendication 1 du composé de formule (I) pour lesquels R₆ représente un groupement alkoxy inférieur ainsi que leurs isomères.

18. Procédé de préparation selon la revendication 1 du composé de formule (I) qui est le Trans (L) 1-(3,4,5-triméthoxy benzoyl) 4-hydroxy pyrolidine-2 N'cyclohexyl méthyl carboxamide, ses isomères trans (D), cis (L), Cis (D).

19. Procédé de préparation selon la revendication 1 du composé de formule (I) qui est le Trans (L) 1-(3,4-diméthoxy benzoyl)4-hydroxy pyrrolidine-2N' cyclooctyl carboxamide, ses isomères Trans (D), cis (L), cis (D).

20. Procédé de préparation selon la revendication 1 du composé de formule (I) qui est le Trans (L) 1-(3,4-diméthoxy benzoyl) 4-hydroxy pyrrolidine-2 carboxamide, ses isomères Trans (D), cis (L), cis (D).

21. Procédé de préparation selon la revendication 1 du composé de formule (I) qui est le Trans (L) 1-(3,4,5-triméthoxy benzoyl) 4-hydroxy pyrrolidine-2 carboxylate d'éthyle, ses isomères Trans (D), cis (L), cis (D).

22. Procédé de préparation de composition pharmaceutique contenant comme principe actif au moins un composé obtenu selon l'une des revendications 1 à 21 en combinaison avec un ou plusieurs excipients ou véhicules inertes non toxiques, pharmaceutiquement acceptables.

23. Procédé de préparation de composition pharmaceutique selon la revendication 22 contenant au moins un principe actif selon l'une des revendications 1 à 21 utilisables dans le traitement des maladies résultant de troubles hypoxiques ischémiques ou oxyprives, les syndromes du déficit intellectuel, la pathologie du sujet âgé, les troubles de l'attention, les infarctus cérébraux constitués et les vertiges d'origine centrale.

## Claims (Claims for the following Contracting State(s): AT, BE, CH, DE, DK, FR, GB, IT, LI, LU, NL, SE)

1. Compounds of the general formula (I): 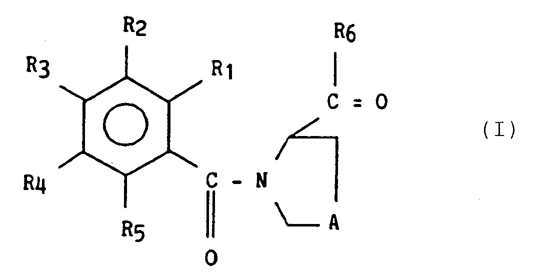 in which:
A represents a group CO or CHOH;
R₁, R₂, R₃, R₄ and R₅, which are the same or different, each independently of the others represents a hydrogen, chlorine or fluorine atom, a lower alkyl group, a cycloalkyl group having from 3 to 8 carbon atoms, a hydroxy group, a lower alkoxy group, a lower alkylamino group, a di-lower alkylamino group, a phenyl-lower alkyl group, a phenyl-lower alkoxy group, a lower alkyl group substituted by one or more halogen atoms, or (and) two adjacent groups from R₁, R₂, R₃, R₄ and R₅, that is to say R₁ and R₂ or R₂ and R₃ or R₃ and R₄ or R₄ and R₅, together form a bridge -O-CH₂-O-, -O-CH₂-CH₂-O- or -O-CH=CH-O-, on the understanding that:
. R₁, R₂, R₃, R₄ and R₅ may not simultaneously represent a hydrogen atom, and
. A may only represent a group C=O when R₁ and R₂ or R₂ and R₃ or R₃ and R₄ or R₄ and R₅ together form a bridge -O-CH₂-O-, -O-CH₂-CH₂-O- or -O-CH=CH-O-;
R₆ represents a hydroxy group of a lower alkoxy group, an amino group or a group NR₇R₈ or -O-B-NR₇R₈, wherein B represents lower alkyl and R₇ and R₈, which are the same or different, represent a hydrogen atom, a lower alkyl group, or cycloalkyl or cycloalkyl-lower alkyl, phenyl, phenyl-lower alkyl, substituted phenyl or substituted phenyl-lower alkyl, or R₇ and R₈ form with the nitrogen atom carrying them a mono- or bi-cyclic heterocyclic system, each ring containing 5 or 6 ring members and optionally including in its skeleton 1 or 2 hetero atoms selected from nitrogen, oxygen and sulphur and being optionally substituted by a lower alkyl group or phenyl or phenyl-lower alkyl, or substituted phenyl-lower alkyl or substituted phenyl, the term substituted associated with the terms phenyl and phenyl-lower alkyl meaning that the aromatic nucleus of those groups may be substituted by one or more groups lower alkyl, lower alkoxy or trifluoromethyl, or R₆ forms with A a lactone system, which confers on the compounds of formula (I) the particular formula (I'): 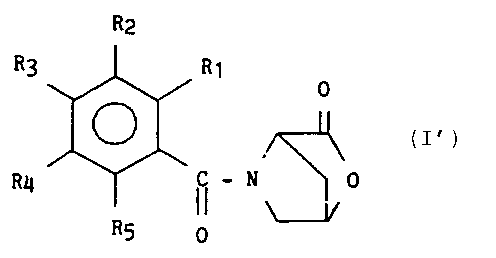 in which R₁, R₂, R₃, R₄ and R₅ are as defined above;
with the provisos that:
. when R₆ represents a hydroxy group and A represents a group CHOH, or R₆ and A together form an internal lactone group (compounds of formula (I')), simultaneously it is not possible to have R₁, R₄ and R₅ each representing simultaneously a hydrogen atom and R₂ and R₃ each representing a chlorine atom and, symmetrically, R₅, R₁ and R₂ representing a hydrogen atom and R₃ and R₄ each representing a chlorine atom,
. when R₆ and A together form an internal lactone group (compounds of formula (I')), at least two of the groups R₁, R₂, R₃, R₄ and R₅ are other than a hydrogen atom,
. R₁ or R₅ may not represent an acetoxy group when simultaneously R₂, R₃, R₄, R₆ and the other group R₁ or R₅ represent a hydrogen atom,
. wherein the term cycloalkyl group is to be understood as meaning groups having from 3 to 8 carbon atoms, and the term lower alkyl group is to be understood as meaning linear or branched groups having from 1 to 6 carbon atoms,
and, where appropriate, their isomers, epimers and diastereoisomers, and addition salts thereof with a pharmaceutically acceptable acid or base.

2. Compounds of formula (I) according to claim 1 in which A represents a group CHOH, their trans-(L) or (2S,4R), trans-(D) or (2R,4S), cis-(L) or (2S,4S) or cis-(D) (2R,4R) isomers and, where appropriate, addition salts thereof with a pharmaceutically acceptable acid or base.

3. Compounds of formula (I) according to claim 1 in which A represents a group CHOH having the trans-(L) or (2S,4R) configuration and, where appropriate, addition salts thereof with a pharmaceutically acceptable acid or base.

4. Compound of formula (I) according to claim 1, characterised in that R₂, R₃ and R₄, which are the same or different, represent a lower alkoxy group or a hydrogen atom or a hydroxy group, while R₁ and R₅ each represent a hydrogen atom, isomers thereof and addition salts thereof with a pharmaceutically acceptable acid or base.

5. Compound according to claim 1 in which R₆ represents a hydroxy group or a lower alkoxy group or an amino group or an N,N-di-lower alkylamino group or 4-(2,3,4-trimethoxybenzyl)-1-piperazinyl, isomers thereof and, where appropriate, addition salts thereof with a pharmaceutically acceptable acid or base.

6. Compound according to claim 1 which is trans-(L)-1-(3,4-dimethoxybenzoyl)-4-hydroxy-2-pyrrolidine-N',N'-diethylcarboxamide, the corresponding carboxylic acid, their trans-(D), cis-(L) and cis-(D) isomers, and addition salts thereof with a pharmaceutically acceptable base.

7. Compound according to claim 1 which is trans-(L)-1-(3,4,5-trimethoxybenzoyl)-4-hydroxy-2-pyrrolidinecarboxylic acid, its trans-(D), cis-(L) and cis-(D) isomers, and addition salts thereof with a pharmaceutically acceptable base.

8. Compound according to claim 1 which is trans-(L)-1-(3,4-dimethoxybenzoyl)-4-hydroxy-2-pyrrolidine-N'-(cyclohexylmethyl)carboxamide, its trans-(D), cis-(L) and cis-(D) isomers.

9. Compound according to claim 1 which is trans-(L)-1-(3,4-dimethoxybenzoyl)-4-hydroxy-2-pyrrolidine-N'-cyclopropylcarboxamide, its trans-(D), cis-(L) and cis-(D) isomers.

10. Compound according to claim 1 which is trans-(L)-1-(3,4-methylenedioxybenzoyl)-4-hydroxy-2-pyrrolidine-N'-(cyclohexylmethyl)carboxamide, its trans-(D), cis-(L) and cis-(D) isomers.

11. Compound according to claim 1 which is 3-(N',N'-dimethylamino)propyl trans-(L)-1-(3,4-methylenedioxybenzoyl)-4-hydroxy-2-pyrrolidinecarboxylate, its trans-(D), cis-(L) and cis-(D) isomers, and addition salts thereof with a pharmaceutically acceptable acid.

12. Compound according to claim 1 which is 2-morpholinoethyl trans-(L)-1-(3,4-methylenedioxybenzoyl)-4-hydroxy-2-pyrrolidinecarboxylate, its trans-(D), cis-(L) and cis-(D) isomers, and addition salts thereof with a pharmaceutically acceptable acid.

13. Compound according to claim 1 which is 2-morpholinoethyl trans-(L)-1-(3,4-dimethoxybenzoyl)-4-hydroxy-2-pyrrolidinecarboxylate, its trans-(D), cis-(L) and cis-(D) isomers, and addition salts thereof with a pharmaceutically acceptable acid.

14. Compound according to claim 1 which is trans-(L)-[4-hydroxy-1-(3,4-methylenedioxybenzoyl)-2-pyrrolidinyl] [4-(2,3,4-trimethoxybenzyl)-1-piperazinyl] ketone, its trans-(D), cis-(L) and cis-(D) isomers, and addition salts thereof with a pharmaceutically acceptable acid.

15. Compound according to claim 1 which is trans-(L)-[4-hydroxy-1-(3,4-dimethoxybenzoyl)-2-pyrrolidinyl] [4-(2,3,4-trimethoxybenzyl)-1-piperazinyl] ketone, its trans-(D), cis-(L) and cis-(D) isomers, and addition salts thereof with a pharmaceutically acceptable acid.

16. Compounds according to claim 1 in which R₆ represents a lower alkoxy group, and their isomers.

17. Compound according to claim 1 which is trans-(L)-1-(3,4,5-trimethoxybenzoyl)-4-hydroxy-2-pyrrolidine-N'-cyclohexylmethylcarboxamide, its trans-(D), cis-(L) and cis-(D) isomers.

18. Compound according to claim 1 which is trans-(L)-1-(3,4-dimethoxybenzoyl)-4-hydroxy-2-pyrrolidine-N'-cyclooctylcarboxamide, its trans-(D), cis-(L) and cis-(D) isomers.

19. Compound according to claim 1 which is trans-(L)-1-(3,4-dimethoxybenzoyl)-4-hydroxy-2-pyrrolidinecarboxamide, its trans-(D), cis-(L) and cis-(D) isomers.

20. Compound according to claim 1 which is ethyl trans-(L)-1-(3,4,5-trimethoxybenzoyl)-4-hydroxy-2-pyrrolidinecarboxylate, its trans-(D), cis-(L) and cis-(D) isomers.

21. Process for the preparation of compounds of the general formula (I) according to claim 1, characterised in that there is used as starting material a compound of formula (II):
in which R₁, R₂, R₃, R₄ and R₅ are as defined in formula (I) and X represents a halogen atom, which is treated with the compound of formula (III): in which R₆ is as defined in formula (I), 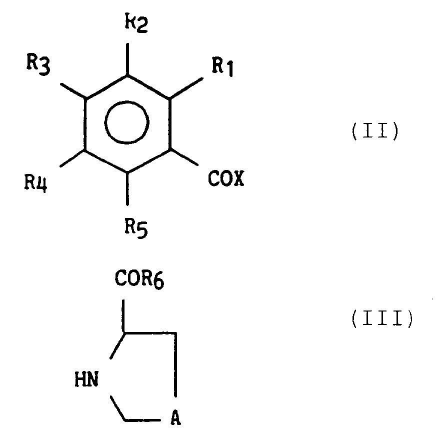 to yield a compound of formula (I): 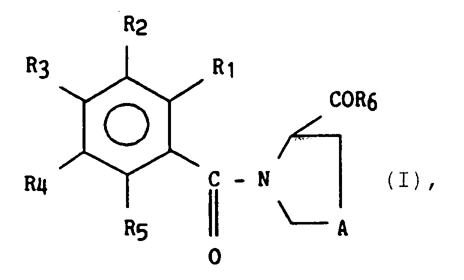 which is separated, where appropriate, into its isomers and which is converted, if desired, into a salt with a pharmaceutically acceptable acid or, where appropriate, base,
which compound of formula (I), when R₆ represents a group OH, can be treated, where appropriate, after optional activation of the carboxylic acid function,
- either with an amine of the formula NR₇R₈ or a compound of the formula X-B-NR₇R₈, wherein X represents a halogen atom and B, R₇ and R₈ are as defined in formula (I), or with a lower aliphatic alcohol R'₆OH, wherein R'₆ represents a lower alkyl group, to obtain a compound of formula (I) in which R₆ represents a group NR₇R₈ or a lower alkyl group, respectively,
- or with imidazole to obtain a compound of formula (I'),
which compound of formula (I) or (I') is purified, if desired, is separated into its isomers, where appropriate, and is converted, if desired, into a salt with a pharmaceutically acceptable acid or base.

22. Process for the preparation of compounds of formula (I) in which A represents a group C=O, characterised in that a compound of formula (I/B): 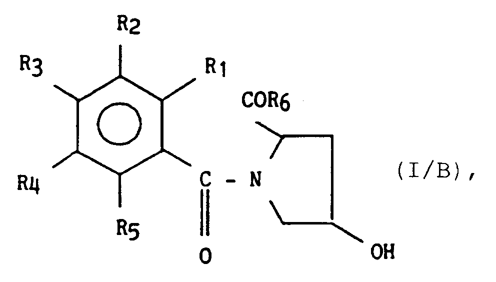 which is a particular case of the compounds of formula (I) in which R₁, R₂, R₃, R₄, R₅ and R₆ are as defined in formula (I) and A represents a group CHOH,
is treated with an oxidising agent to obtain a compound of formula (I/C): 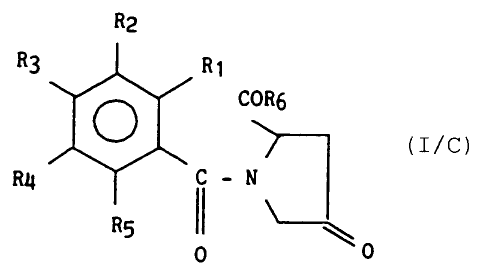 in which R₁, R₂, R₃, R₄, R₅ and R₆ are as defined in formula (I),
which is purified and which is converted, if desired, into a salt with a pharmaceutically acceptable acid or base, as appropriate,
and which can be treated, when R₆ represents a hydroxy group, depending on the compound of formula (I) that is to be obtained, either with an amine of the formula NR₇R₈ or a compound X-B-NR₇R₈, wherein B, R₇ and R₈ are as defined in formula (I) and X represents a halogen atom, or with a lower aliphatic alcohol R'₆-OH, wherein R'₆ represents a lower alkyl group, to obtain a compound of formula (I) in which R₆ represents a group NR₇R₈ or a lower alkyl group, respectively,
which is purified, if necessary, and the isomers of which are separated, where appropriate, and which is converted, if desired, into a salt with a pharmaceutically acceptable acid or base, as appropriate.

23. Pharmaceutical composition comprising as active ingredient at least one compound according to one of claims 1 to 20, in combination with one or more inert, non-toxic, pharmaceutically acceptable excipients or carriers.

24. Pharmaceutical composition according to claim 23 comprising at least one active ingredient according to one of claims 1 to 20 for use in the treatment of diseases resulting from hypoxic or ischaemic disorders or disorders of oxygen deprivation, syndromes of mental deficiency, the pathology of ageing, attention deficiency disorders, constitutional cerebral infarct and vertigos of central origin.

## Claims (Claims for the following Contracting State(s): ES, GR)

1. Process for the preparation of compounds of the general formula (I): 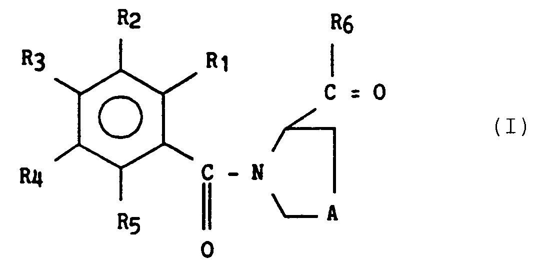 in which:
A represents a group CO or CHOH;
R₁, R₂, R₃, R₄ and R₅, which are the same or different, each independently of the others represents a hydrogen, chlorine or fluorine atom, a lower alkyl group, a cycloalkyl group having from 3 to 8 carbon atoms, a hydroxy group, a lower alkoxy group, a lower alkylamino group, a di-lower alkylamino group, a phenyl-lower alkyl group, a phenyl-lower alkoxy group, a lower alkyl group substituted by one or more halogen atoms, or (and) two adjacent groups from R₁, R₂, R₃, R₄ and R₅, that is to say R₁ and R₂ or R₂ and R₃ or R₃ and R₄ or R₄ and R₅, together form a bridge -O-CH₂-O-, -O-CH₂-CH₂-O- or -O-CH=CH-O-, on the understanding that:
. R₁, R₂, R₃, R₄ and R₅ may not simultaneously represent a hydrogen atom, and
. A may only represent a group C=O when R₁ and R₂ or R₂ and R₃ or R₃ and R₄ or R₄ and R₅ together form a bridge -O-CH₂-O-, -O-CH₂-CH₂-O- or -O-CH=CH-O-;
R₆ represents a hydroxy group or a lower alkoxy group, an amino group or a group NR₇R₈ or -O-B-NR₇R₈, wherein B represents lower alkyl and R₇ and R₈, which are the same or different, represent a hydrogen atom, a lower alkyl group, or cycloalkyl or cycloalkyl-lower alkyl, phenyl, phenyl-lower alkyl, substituted phenyl or substituted phenyl-lower alkyl, or R₇ and R₈ form with the nitrogen atom carrying them a mono- or bi-cyclic heterocyclic system, each ring containing 5 or 6 ring members and optionally including in its skeleton 1 or 2 hetero atoms selected from nitrogen, oxygen and sulphur and being optionally substituted by a lower alkyl group or phenyl or phenyl-lower alkyl, or substituted phenyl-lower alkyl or substituted phenyl, the term substituted associated with the terms phenyl and phenyl-lower alkyl meaning that the aromatic nucleus of those groups may be substituted by one or more groups lower alkyl, lower alkoxy or trifluoromethyl, or R₆ forms with A a lactone system, which confers on the compounds of formula (I) the particular formula (I'): 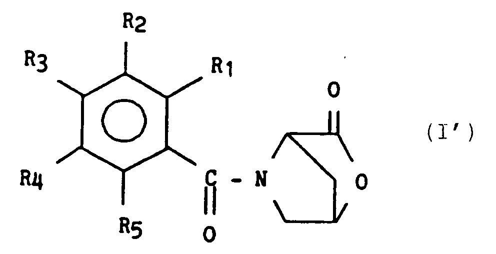 in which R₁, R₂, R₃, R₄ and R₅ are as defined above;
with the provisos that:
. when R₆ represents a hydroxy group and A represents a group CHOH, or R₆ and A together form an internal lactone group (compounds of formula (I')), simultaneously it is not possible to have R₁, R₄ and R₅ each representing simultaneously a hydrogen atom and R₂ and R₃ each representing a chlorine atom and, symmetrically, R₅ R₁ and R₂ representing a hydrogen atom and R₃ and R₄ each representing a chlorine atom,
. when R₆ and A together form an internal lactone group (compounds of formula (I')), at least two of the groups R₁, R₂, R₃, R₄ and R₅ are other than a hydrogen atom,
. R₁ or R₅ may not represent an acetoxy group when simultaneously R₂, R₃, R₄, R₆ and the other group R₁ or R₅ represent a hydrogen atom,
. wherein the term cycloalkyl group is to be understood as meaning groups having from 3 to 8 carbon atoms, and the term lower alkyl group is to be understood as meaning linear or branched groups having from 1 to 6 carbon atoms,
and, where appropriate, their isomers, epimers and diastereoisomers, and addition salts thereof with a pharmaceutically acceptable acid or base,
characterised in that there is used as starting material a compound of formula (II): 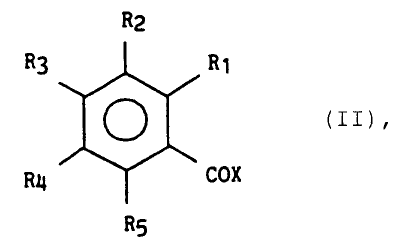 in which R₁, R₂, R₃, R₄ and R₅ are as defined in formula (I) and X represents a halogen atom,
which is treated with the compound of formula (III): 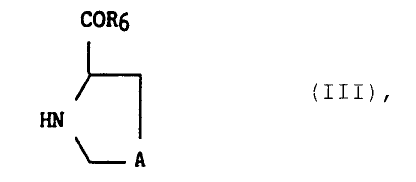 in which R₆ is as defined in formula (I),
to yield a compound of formula (I): 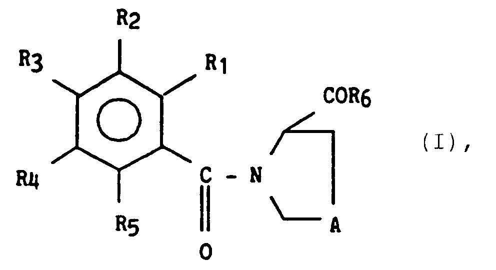 which is separated, where appropriate, into its isomers and which is converted, if desired, into a salt with a pharmaceutically acceptable acid or, where appropriate, base,
which compound of formula (I), when R₆ represents a group OH, can be treated, where appropriate, after optional activation of the carboxylic acid function,
- either with an amine of the formula NR₇R₈ or a compound of the formula X-B-NR₇R₈, wherein X represents a halogen atom and B, R₇ and R₈ are as defined in formula (I), or with a lower aliphatic alcohol R'₆OH, wherein R'₆ represents a lower alkyl group, to obtain a compound of formula (I) in which R₆ represents a group NR₇R₈ or a lower alkyl group, respectively,
- or with imidazole to obtain a compound of formula (I'),
which compound of formula (I) or (I') is purified, if desired, is separated into its isomers, where appropriate, and is converted, if desired, into a salt with a pharmaceutically acceptable acid or base.

2. Process for the preparation of compounds of formula (I) in which A represents a group C=O, characterised in that a compound of formula (I/B): 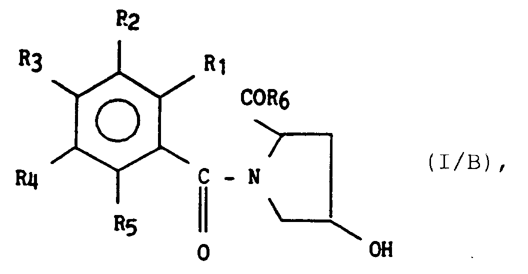 which is a particular case of the compounds of formula (I) in which R₁, R₂, R₃, R₄, R₅ and R₆ are as defined in formula (I) and A represents a group CHOH,
is treated with an oxidising agent to obtain a compound of formula (I/C): 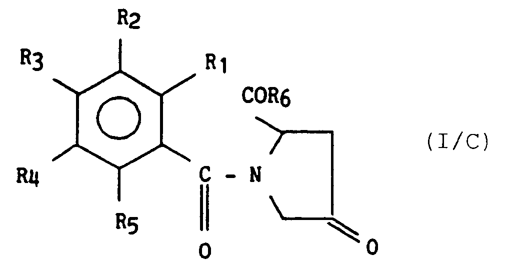 in which R₁, R₂, R₃, R₄, R₅ and R₆ are as defined in formula (I),
which is purified and which is converted, if desired, into a salt with a pharmaceutically acceptable acid or base, as appropriate,
and which can be treated, when R₆ represents a hydroxy group, depending on the compound of formula (I) that is to be obtained, either with an amine of the formula NR₇R₈ or a compound X-B-NR₇R₈, wherein B, R₇ and R₈ are as defined in formula (I) and X represents a halogen atom, or with a lower aliphatic alcohol R'₆OH, wherein R'₆ represents a lower alkyl group, to obtain a compound of formula (I) in which R₆ represents a group NR₇R₈ or a lower alkyl group, respectively,
which is purified, if necessary, and the isomers of which are separated, where appropriate, and which is converted, if desired, into a salt with a pharmaceutically acceptable acid or base, as appropriate.

3. Process according to claim 1 for the preparation of compounds of formula (I) in which A represents a group CHOH, their trans-(L) or (2S,4R), trans-(D) or (2R,4S), cis-(L) or (2S,4S) or cis-(D) (2R,4R) isomers and, where appropriate, addition salts thereof with a pharmaceutically acceptable acid or base.

4. Process according to claim 1 for the preparation of compounds of formula (I) in which A represents a group CHOH having the trans-(L) or (2S,4R) configuration and, where appropriate, addition salts thereof with a pharmaceutically acceptable acid or base.

5. Process according to claim 1 for the preparation of compounds of formula (I) according to claim 1, characterised in that R₂, R₃ and R₄, which are the same or different, represent a lower alkoxy group or a hydrogen atom or a hydroxy group, while R₁ and R₅ each represent a hydrogen atom, isomers thereof and addition salts thereof with a pharmaceutically acceptable acid or base.

6. Process according to claim 1 for the preparation of the compound of formula (I) in which R₆ represents a hydroxy group or a lower alkoxy group or an amino group or an N,N-di-lower alkylamino group or 4-(2,3,4-trimethoxybenzyl)-1-piperazinyl, isomers thereof and, where appropriate, addition salts thereof with a pharmaceutically acceptable acid or base.

7. Process according to claim 1 for the preparation of the compound of formula (I) which is trans-(L)-1-(3,4-dimethoxybenzoyl)-4-hydroxy-2-pyrrolidine-N',N'-diethylcarboxamide, the corresponding carboxylic acid, their trans-(D), cis-(L) and cis-(D) isomers, and addition salts thereof with a pharmaceutically acceptable base.

8. Process according to claim 1 for the preparation of the compound of formula (I) which is trans-(L)-1-(3,4,5-trimethoxybenzoyl)-4-hydroxy-2-pyrrolidinecarboxylic acid, its trans-(D), cis-(L) and cis-(D) isomers, and addition salts thereof with a pharmaceutically acceptable base.

9. Process according to claim 1 for the preparation of the compound of formula (I) which is trans-(L)-1-(3,4-dimethoxybenzoyl)-4-hydroxy-2-pyrrolidine-N'-(cyclohexylmethyl)carboxamide, its trans-(D), cis-(L) and cis-(D) isomers.

10. Process according to claim 1 for the preparation of the compound of formula (I) which is trans-(L)-1-(3,4-dimethoxybenzoyl)-4-hydroxy-2-pyrrolidine-N'-cyclopropylcarboxamide, its trans-(D), cis-(L) and cis-(D) isomers.

11. Process according to claim 1 for the preparation of the compound of formula (I) which is trans-(L)-1-(3,4-methylenedioxybenzoyl)-4-hydroxy-2-pyrrolidine-N'-(cyclohexylmethyl)carboxamide, its trans-(D), cis-(L) and cis-(D) isomers.

12. Process according to claim 1 for the preparation of the compound of formula (I) which is 3-(N',N'-dimethylamino)propyl trans-(L)-1-(3,4-methylenedioxybenzoyl)-4-hydroxy-2-pyrrolidinecarboxylate, its trans-(D), cis-(L) and cis-(D) isomers, and addition salts thereof with a pharmaceutically acceptable acid.

13. Process according to claim 1 for the preparation of the compound of formula (I) which is 2-morpholinoethyl trans-(L)-1-(3,4-methylenedioxybenzoyl)-4-hydroxy-2-pyrrolidinecarboxylate, its trans-(D), cis-(L) and cis-(D) isomers, and addition salts thereof with a pharmaceutically acceptable acid.

14. Process according to claim 1 for the preparation of the compound of formula (I) which is 2-morpholinoethyl trans-(L)-1-(3,4-dimethoxybenzoyl)-4-hydroxy-2-pyrrolidinecarboxylate, its trans-(D), cis-(L) and cis-(D) isomers, and addition salts thereof with a pharmaceutically acceptable acid.

15. Process according to claim 1 for the preparation of the compound of formula (I) which is trans-(L)-[4-hydroxy-1-(3,4-methylenedioxybenzoyl)-2-pyrrolidinyl] [4-(2,3,4-trimethoxybenzyl)-1-piperazinyl] ketone, its trans-(D), cis-(L) and cis-(D) isomers, and addition salts thereof with a pharmaceutically acceptable acid.

16. Process according to claim 1 for the preparation of the compound of formula (I) which is trans-(L)-[4-hydroxy-1-(3,4-dimethoxybenzoyl)-2-pyrrolidinyl] [4-(2,3,4-trimethoxybenzyl)-1-piperazinyl] ketone, its trans-(D), cis-(L) and cis-(D) isomers, and addition salts thereof with a pharmaceutically acceptable acid.

17. Process according to claim 1 for the preparation of the compounds of formula (I) in which R₆ represents a lower alkoxy group, and their isomers.

18. Process according to claim 1 for the preparation of the compound of formula (I) which is trans-(L)-1-(3,4,5-trimethoxybenzoyl)-4-hydroxy-2-pyrrolidine-N'-cyclohexylmethylcarboxamide, its trans-(D), cis-(L) and cis-(D) isomers.

19. Process according to claim 1 for the preparation of the compound of formula (I) which is trans-(L)-1-(3,4-dimethoxybenzoyl)-4-hydroxy-2-pyrrolidine-N'-cyclooctylcarboxamide, its trans-(D), cis-(L) and cis-(D) isomers.

20. Process according to claim 1 for the preparation of the compound of formula (I) which is trans-(L)-1-(3,4-dlmethoxybenzoyl)-4-hydroxy-2-pyrrolidinecarboxamide, its trans-(D), cis-(L) and cis-(D) isomers.

21. Process according to claim 1 for the preparation of the compound of formula (I) which is ethyl trans-(L)-1-(3,4,5-trimethoxybenzoyl)-4-hydroxy-2-pyrrolidinecarboxylate, its trans-(D), cis-(L) and cis-(D) isomers.

22. Process for the preparation of a pharmaceutical composition comprising as active ingredient at least one compound obtained according to one of claims 1 to 21, in combination with one or more inert, non-toxic, pharmaceutically acceptable excipients or carriers.

23. Process according to claim 22 for the preparation of a pharmaceutical composition comprising at least one active ingredient according to one of claims 1 to 21, for use in the treatment of diseases resulting from hypoxic or ischaemic disorders or disorders of oxygen deprivation, syndromes of mental deficiency, the pathology of ageing, attention deficiency disorders, constitutional cerebral infarct and vertigos of central origin.

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel (I): 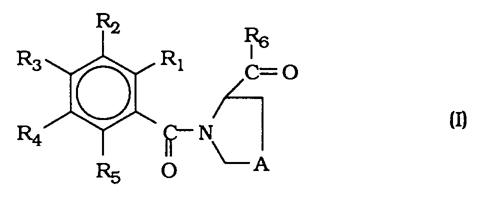 in der
A eine Gruppe CO oder CHOH bedeutet,
R₁, R₂, R₃, R₄ und R₅, die gleichartig oder verschieden sein können, unabhängig voneinander jeweils ein Wasserstoffatom, ein Chloratom oder ein Fluoratom, eine Niedrigalkylgruppe, eine Cycloalkylgruppe mit 3 bis 8 Kohlenstoffatomen, eine Hydroxylgruppe, eine Niedrigalkoxygruppe, eine Niedrigalkylaminogruppe, eine Niedrigdialkylaminogruppe, eine Phenylniedrigalkylgruppe, eine Phenylniedrigalkyloxygruppe, eine Niedrigalkylgruppe, die durch eines oder mehrere Halogenatome substituiert ist, und/oder zwei benachbarte der Gruppen R₁, R₂, R₃, R₄ und R₅, d. h. R₁ und R₂ oder R₂ und R₃ oder R₃ und R₄ oder R₄ und R₅, gemeinsam eine Brücke -O-CH₂-O-, -O-CH₂-CH₂-O- oder -O-CH=CH-O- bilden, bedeuten, mit der Maßgabe, daß:
. R₁, R₂, R₃, R₄ und R₅ nicht gleichzeitig ein Wasserstoffatom darstellen,
. A keine Gruppe C=O darstellt, wenn R₁ und R₂ oder R₂ und R₃ oder R₃ und R₄ oder R₄ und R₅ gemeinsam eine Brücke -O-CH₂-O- oder -O-CH₂-CH₂-O- oder -O-CH=CH-O- darstellen, und
R₆ eine Hydroxylgruppe oder eine Niedrigalkoxygruppe, eine Aminogruppe oder eine Gruppe NR₇R₈ oder -O-B-NR₇R₈, worin B eine Niedrigalklgruppe und R₇ und R₈, die gleichartig oder verschieden sein können jeweils ein Wasserstoffatom, eine Niedrigalkylgruppe, eine Cycloalkylgruppe oder eine Cycloalkylniedrigalklgruppe, eine Phenylgruppe, eine Phenylniedrig-alkylgruppe, eine substituierte Phenylgruppe oder eine substituierte Phenylniedrigalkylgruppe darstellen, oder R₇ und R₈ mit dem Stickstoffatom, das sie trägt, ein monocyclisches oder bicyclisches heterocyclisches System bilden, wobei jeder Ring fünf bis sechs Ringglieder aufweist und gegebenenfalls in seinem Gerüst ein bis zwei Heteroatome ausgewählt aus Stickstoff, Sauerstoff oder Schwefel aufweist und gegebenenfalls durch eine Niedrigalkylgruppe, eine Phenylgruppe oder eine Phenylniedrigalkylgruppe oder eine substituierte Phenylniedrigalkylgruppe oder eine substituierte Phenylgruppe substituiert ist, wobei der sich auf die Phenylgruppe und Phenylniedrigalkylgruppe beziehende Ausdruck "substituiert" bedeutet, daß der aromatische Kern dieser Gruppen durch eine oder mehrere Niedrigalkylgruppen, Niedrigalkoxygruppen oder Trifluormethylgruppen substituiert sein kann, bedeutet, oder R₆ mit A ein Lactonsystem bildet, welches den Derivaten der Formel (I) die besondere Formel (I') verleiht: 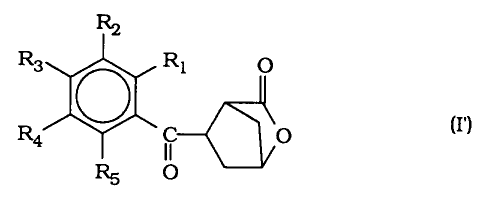 in der R₁, R₂, R₃, R₄ und R₅ die oben angegebenen Bedeutungen besitzen, mit der Maßgabe, daß:
. wenn R₆ eine Hydroxylgruppe und A eine Gruppe CHOH bedeuten oder R₆ und A gemeinsam eine interne Lactongruppe bilden (Derivate der Formel (I')), R₁, R₄ und R₅ nicht gleichzeitigjeweils ein Wasserstoffatom bedeuten und R₂ und R₃ jeweils ein Chloratom darstellen und symmetrisch R₅, R₁ und R₂ ein Wasserstoffatom und R₃ und R₄ jeweils ein Chloratom bedeuten,
. wenn R₆ und A gemeinsam eine interne Lactongruppe darstellen (Derivate der Formel (I')), mindestens zwei der Gruppen R₁, R₂, R₃, R₄ und R₅ von dem Wasserstoffatom verschieden sind,
. R₁ oder R₅ keine Acetyloxygruppe darstellen, wenn gleichzeitig R₂, R₃, R₄, R₆ und die andere der Gruppen R₁ und R₅ ein Wasserstoffatom darstellen,
. wobei es sich versteht, daß unter Cycloalkylgruppen Gruppen mit 3 bis 8 Kohlenstoffatomen und unter Niedrigalkylgruppen geradkettige oder verzweigte Gruppen mit 1 bis 6 Kohlenstoffatomen zu verstehen sind,
sowie gegebenenfalls deren Isomere, Epimere, Diastereoisomere und deren Additionssalze mit einer pharmazeutisch annehmbaren Säure oder Base.

2. Verbindungen der Formel (I) nach Anspruch 1, worin A eine Gruppe CHOH darstellt, deren trans-(L)- oder (2S,4R)-Isomeren, trans-(-D)- oder (2R,4S)-Isomeren, cis-(L)- oder (2S,4S)-Isomeren oder cis-(D)- oder (2R,4R)-Isomeren sowie gegebenenfalls deren Additionssalze mit einer pharmazeutisch annehmbaren Säure oder Base.

3. Verbindungen der Formel (I) nach Anspruch 1, worin A eine Gruppe CHOH darstellt, der Konfiguration trans-(L) oder (2S,4R) und gegebenenfalls deren Additionssalze mit einer pharmazeutisch annehmbaren Säure oder Base.

4. Verbindung der Formel (I) nach Anspruch 1, **dadurch gekennzeichnet**, daß R₂, R₃ und R₄, die gleichartig oder verschieden sein können,jeweils eine Niedrigalkoxygruppe oder ein Wasserstoffatom oder eine Hydroxylgruppe bedeuten, während R₁ und R₅ jeweils ein Wasserstoffatom darstellen, deren Isomere sowie deren Additionssalze mit einer pharmazeutisch annehmbaren Säure oder Base.

5. Verbindung nach Anspruch 1, worin R₆ eine Hydroxylgruppe oder eine Niedrigalkoxygruppe oder eine Aminogruppe oder eine N,N-Niedrigdialkylaminogruppe oder eine 4-(2,3,4-Trimethoxy-benzyl)piperazin-1-yl-gruppe bedeutet, deren Isomere sowie gegebenenfalls deren Additionssalze mit einer pharmazeutisch annehmbaren Säure oder Base.

6. Verbindung nach Anspruch 1, nämlich trans-(L)-1-(3,4-Dimethoxy-benzoyl)-4-hydroxy-pyrrolidin-2-N',N'diethylcarboxamid, dessen entsprechende Carbonsäure und deren trans-(D)-, cis-(L)- und cis(D)-Isomere sowie deren Additionssalze mit einer pharmazeutisch annehmbaren Base.

7. Verbindung nach Anspruch 1, nämlich trans-(L)-1-(3,4,5-Trimethoxy-benzoyl)-4-hydroxy-pyrrolidin-2-carbonsäure, deren trans-(D)-, cis-(L)- und cis-(D)-Isomere sowie deren Additionssalze mit einer pharmazeutisch annehmbaren Base.

8. Verbindung nach Anspruch 1, nämlich trans-(L)-1-(3,4-Dimethoxy-benzoyl)-4-hydroxy-pyrrolidin-2-N'-(cyclohexylmethyl)-carboxamid und dessen trans-(D)-, cis-(L)- und cis-(D)-Isomere.

9. Verbindung nach Anspruch 1, nämlich trans-(L)-1-(3,4-Dimethoxy-benzoyl)-4-hydroxy-pyrrolidin-2-N'-cyclopropylcarboxamid und dessen trans-(D)-, cis-(L)- und cis-(D)-Isomere.

10. Verbindung nach Anspruch 1, nämlich trans-(L)-1-(3,4-Methylen-dioxybenzoyl)-4-hydroxy-pyrrolidin-2-N'-(cyclohexylmethyl)-carboxamid und dessen trans-(D)-, cis-(L)- und cis-(D)-Isomere.

11. Verbindung nach Anspruch 1, nämlich trans-(L)-1-(3,4-Methylendioxybenzoyl)-4-hydroxy-pyrrolidin-2-carbonsäure-3-(N',N'-dimethylamino)-propylester und dessen trans-(D)-, cis-(L)- und cis-(D)-Isomere sowie deren Additionssalze mit einer pharmazeutisch annehmbaren Säure.

12. Verbindung nach Anspruch 1, nämlich trans-(L)-1-(3,4-Methylendioxybenzoyl)-4-hydroxy-pyrrolidin-2-carbonsäure-2-morpholinoethylester und dessen trans-(D)-, cis-(L)- und cis-(D)-Isomere sowie deren Additionssalze mit einer pharmazeutisch annehmbaren Säure.

13. Verbindung nach Anspruch 1, nämlich trans-(L)-1-(3,4-Dimethoxybenzoyl)-4-hydroxy-pyrrolidin-2-carbonsäure-2-morpholinoethylester und dessen trans-(D)-, cis-(L)- und cis-(D)-Isomere sowie deren Additionssalze mit einer pharmazeutisch annehmbaren Säure.

14. Verbindung nach Anspruch 1, nämlich trans-(L)-[4-Hydroxy-1-(3,4-methylendioxybenzoyl)-pyrrolidin-2-yl]-[4-(2,3,4-trimethoxybenzyl)-piperazin-1-yl)-keton und dessen trans-(D)-, cis-(L)- und cis-(D)-Isomere sowie deren Additionssalze mit einer pharmazeutisch annehmbaren Säure.

15. Verbindung nach Anspruch 1, nämlich trans-(L)-[4-Hydroxy-1-(3,4-Dimethoxybenzoyl)-pyrrolidin-2-yl]-[4-(2,3,4-trimethoxybenzyl)-piperazin-1-yl)-keton und dessen trans-(D)-, cis-(L)- und cis-(D)-Isomere sowie deren Additionssalze mit einer pharmazeutisch annehmbaren Säure.

16. Verbindungen nach Anspruch 1, worin R₆ eine Niedrigalkoxygruppe darstellt sowie deren Isomere.

17. Verbindung nach Anspruch 1, nämlich trans-(L)-1-(3,4,5-Trimethoxybenzoyl)-4-hydroxy-pyrrolidin-2-N'-cyclohexylmethylcarboxamid und dessen trans-(D)-, cis-(L)- und cis-(D)-Isomere.

18. Verbindung nach Anspruch 1, nämlich trans-(L)-1-(3,4-Dimethoxybenzoyl)-4-hydroxy-pyrrolidin-2-N'-cyclooctylcarboxamid und dessen trans-(D)-, cis-(L)- und cis-(D)-Isomere.

19. Verbindung nach Anspruch 1, nämlich trans-(L)-1-(3,4-Dimethoxybenzoyl)-4-hydroxy-pyrrolidin-2-carboxamid und dessen trans-(D)-, cis-(L)- und cis-(D)-Isomere.

20. Verbindung nach Anspruch 1, nämlich trans-(L)-1-(3,4,5-Trimethoxybenzoyl)-4-hydroxy-pyrrolidin-2-carbonsäureethylester und dessen trans-(D)-, cis-(L)- und cis-(D)-Isomere.

21. Verfahren zur Herstellung der Verbindungen der allgemeinen Formel (I) nach Anspruch 1, **dadurch gekennzeichnet**, daß man als Ausgangsmaterial ein Derivat der Formel (II) verwendet: 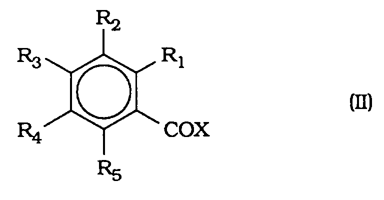 in der R₁, R₂, R₃, R₄ und R₅ die bezüglich der Formel (I) angegebenen Bedeutungen besitzen und X ein Halogenatom darstellt, welches man mit dem Derivat der Formel (III): 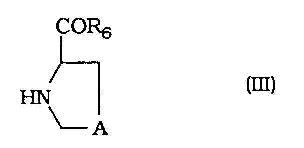 in der R₆ die bezüglich der Formel (I) angegebenen Bedeutungen besitzt, behandelt zur Bildung eines Derivats der Formel (I): 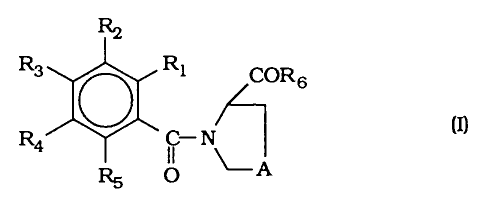 welches man gegebenenfalls in seine Isomeren auftrennt und gewünschtenfalls mit einer Säure oder gegebenenfalls mit einer pharmazeutisch annehmbaren Base in sein Salz überführt, welches Derivat der Formel (I), wenn R₆ eine Gruppe OH darstellt, gegebenenfalls nach der eventuellen Aktivierung der Carbonsäurefunktion
- entweder mit einem Amin der Formel NR₇R₈ oder einem Derivat der Formel X-B-NR₇R₈, worin X ein Halogenatom darstellt, und B, R₇ und R₈ die bezüglich der Formel (I) angegebenen Bedeutungen besitzen, oder mit einem niedrigen aliphatischen Alkohol R'₆OH, worin R'₆ eine Niedrigalkylgruppe darstellt, umsetzt zur Bildung einer Verbindung der Formel (I), worin R₆ eine Gruppe NR₇R₈ bzw. eine Niedrigalkylgruppe darstellt,
- oder mit Imidazol behandelt, so daß man ein Derivat der Formel (I') erhält, welches Derivat der Formel (I) oder (I') man gewünschtenfalls reinigt, gegebenenfalls in seine Isomeren auftrennt und gewünschtenfalls mit einer pharmazeutisch annehmbaren Säure oder Base in ein Salz überführt.

22. Verfahren zur Herstellung der Derivate der Formel (I), worin A eine Gruppe C=O darstellt, **dadurch gekennzeichnet**, daß man ein Derivat der Formel (I/B): 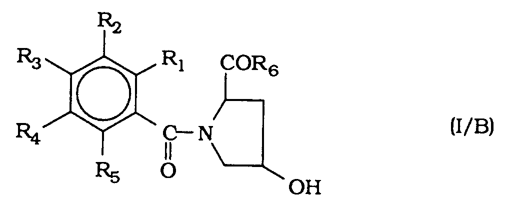 einem Sonderfall der Derivate der Formel (I), worin R₁, R₂, R₃, R₄, R₅ und R₆ die bezüglich der Formel (I) angegebenen Bedeutungen besitzen und A eine Gruppe CHOH darstellt,
mit einem Oxidationsmittel behandelt, so daß man ein Derivat der Formel (I/C) erhält: 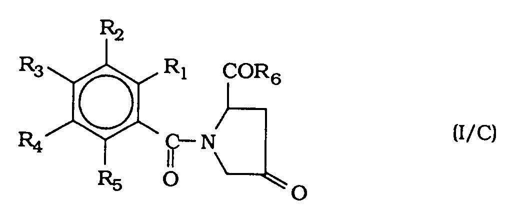 in der R₁, R₂, R₃, R₄, R₅ und R₆ die bezüglich der Formel (I) angegebenen Bedeutungen besitzen,
welches man reinigt und gewünschtenfalls je nachdem mit einer pharmazeutisch annehmbaren Säure oder Base in ein Salz überführt,
und welches man, wenn R₆ eine Hydroxylgruppe darstellt, in Abhängigkeit von dem Derivat der Formel (I), welches man herzustellen wünscht, entweder mit einem Amin der Formel NR₇R₈ oder einem Derivat X-B-NR₇R₈, worin B, R₇ und R₈ die bezüglich der Formel (I) angegebenen Bedeutungen besitzen und X ein Halogenatom darstellt, oder mit einem niedrigaliphatischen Alkohol R'₆OH, worin R'₆ eine Niedrigalkylgruppe bedeutet, behandeln kann, so daß man eine Verbindung der Formel (I) erhält, worin R₆ eine Gruppe NR₇R₈ bzw. eine Niedrigalkylgruppe darstellt,
welche man erforderlichenfalls reinigt und gegebenenfalls in die Isomeren auftrennt und man gewünschtenfalls je nachdem mit einer pharmazeutisch annehmbaren Säure oder Base in ein Salz überführt.

23. Pharmazeutische Zubereitung enthaltend als Wirkstoff mindestens eine Verbindung nach einem der Ansprüche 1 bis 20 in Kombination mit einem oder mehreren inerten, nichttoxischen, pharmazeutisch annehmbaren Trägermaterialien oder Bindemitteln.

24. Pharmazeutische Zubereitung nach Anspruch 23, enthaltend mindestens einen Wirkstoff nach einem der Ansprüche 1 bis 20 zur Verwendung bei der Behandlung von Erkrankungen, die sich durch ischämische hypoxische Störungen oder Sauerstoffversorgungsstörungen ergeben, Syndromen von intellektuellem Defizit, pathologischen Zuständen des Alterns, Aufmerksamkeitsstörungen, konstituierten Zerebralinfarkten und Schwindelzuständen zentralen Ursprungs geeignet sind.

## Patentansprüche (Patentansprüche für folgende(n) Vertragsstaat(en): ES, GR)

1. Verfahren zur Herstellung der Verbindungen der allgemeinen Formel (I): 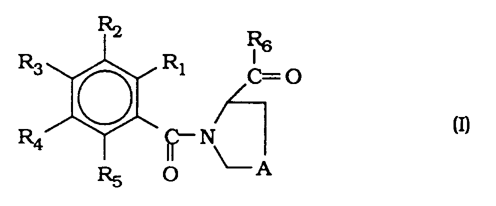 in der
A eine Gruppe CO oder CHOH bedeutet,
R₁, R₂, R₃, R₄ und R₅, die gleichartig oder verschieden sein können, unabhängig voneinander jeweils ein Wasserstoffatom, ein Chloratom oder ein Fluoratom, eine Niedrigalkylgruppe, eine Cycloalkylgruppe mit 3 bis 8 Kohlenstoffatomen, eine Hydroxylgruppe, eine Niedrigalkoxygruppe, eine Niedrigalkylaminogruppe, eine Niedrigdialkylaminogruppe, eine Phenylniedrigalkylgruppe, eine Phenylniedrigalkyloxygruppe, eine Niedrigalkylgruppe, die durch eines oder mehrere Halogenatome substituiert ist, und/ oder zwei benachbarte der Gruppen R₁, R₂, R₃, R₄ und R₅, d. h. R₁ und R₂ oder R₂ und R₃ oder R₃ und R₄ oder R₄ und R₅, gemeinsam eine Brücke -O-CH₂-O-, -O-CH₂-CH₂-O- oder -O-CH=CH-O- bilden, bedeuten, mit der Maßgabe, daß:
. R₁, R₂, R₃, R₄ und R₅ nicht gleichzeitig ein Wasserstoffatom darstellen,
. A keine Gruppe C=O darstellt, wenn R₁ und R₂ oder R₂ und R₃ oder R₃ und R₄ oder R₄ und R₅ gemeinsam eine Brücke -O-CH₂-O- oder -O-CH₂-CH₂-O- oder -O-CH=CH-O- darstellen, und
R₆ eine Hydroxylgruppe oder eine Niedrigalkoxygruppe, eine Aminogruppe oder eine Gruppe NR₇R₈ oder -O-B-NR₇R₈, worin B eine Niedrigalkylgruppe und R₇ und R₈, die gleichartig oder verschieden sein können, jeweils ein Wasserstoffatom, eine Niedrigalkylgruppe, eine Cycloalkylgruppe oder eine Cycloalkylniedrigalkylgruppe, eine Phenylgruppe, eine Phenylniedrig-alkylgruppe, eine substituierte Phenylgruppe oder eine substituierte Phenylniedrigalkylgruppe darstellen, oder R₇ und R₈ mit dem Stickstoffatom, das sie trägt, ein monocyclisches oder bicyclisches heterocyclisches System bilden, wobei jeder Ring fünf bis sechs Ringglieder aufweist und gegebenenfalls in seinem Gerüst ein bis zwei Heteroatome ausgewählt aus Stickstoff, Sauerstoff oder Schwefel aufweist und gegebenenfalls durch eine Niedrigalkylgruppe, eine Phenylgruppe oder eine Phenylniedrigalkylgruppe oder eine substituierte Phenylniedrigalkylgruppe oder eine substituierte Phenylgruppe substituiert ist, wobei der sich auf die Phenylgruppe und Phenylniedrigalkylgruppe beziehende Ausdruck "substituiert" bedeutet, daß der aromatische Kern dieser Gruppen durch eine oder mehrere Niedrigalkylgruppen, Niedrigalkoxygruppen oder Trifluormethylgruppen substituiert sein kann, bedeutet, oder R₆ mit A ein Lactonsystem bildet, welches den Derivaten der Formel (I) die besondere Formel (I') verleiht: 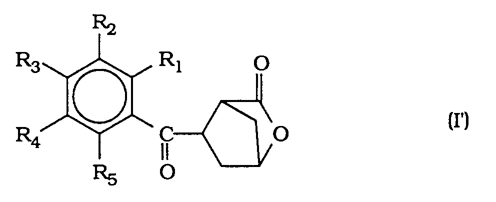 in der R₁, R₂, R₃, R₄ und R₅ die oben angegebenen Bedeutungen besitzen, mit der Maßgabe, daß:
. wenn R₆ eine Hydroxylgruppe und A eine Gruppe CHOH bedeuten oder R₆ und A gemeinsam eine interne Lactongruppe bilden (Derivate der Formel (I')), R₁, R₄ und R₅ nicht gleichzeitig jeweils ein Wasserstoffatom bedeuten und R₂ und R₃ jeweils ein Chloratom darstellen und symmetrisch R₅, R₁ und R₂ ein Wasserstoffatom und R₃ und R₄ jeweils ein Chloratom bedeuten,
. wenn R₆ und A gemeinsam eine interne Lactongruppe darstellen (Derivate der Formel (I')), mindestens zwei der Gruppen R₁, R₂, R₃, R₄ und R₅ von dem Wasserstoffatom verschieden sind,
. R₁ oder R₅ keine Acetyloxygruppe darstellen, wenn gleichzeitig R₂, R₃, R₄, R₆ und die andere der Gruppen R₁ und R₅ ein Wasserstoffatom darstellen,
. wobei es sich versteht, daß unter Cycloalkylgruppen Gruppen mit 3 bis 8 Kohlenstoffatomen und unter Niedrigalkylgruppen geradkettige oder verzweigte Gruppen mit 1 bis 6 Kohlenstoffatomen zu verstehen sind,
sowie gegebenenfalls von deren Isomeren, Epimeren, Diastereoisomeren und deren Additionssalzen mit einer pharmazeutisch annehmbaren Säure oder Base, **dadurch gekennzeichnet**, daß man als Ausgangsmaterial ein Derivat der Formel (II) verwendet: 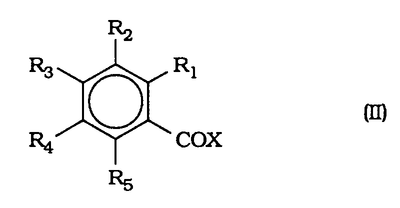 in der R₁, R₂, R₃, R₄ und R₅ die bezüglich der Formel (I) angegebenen Bedeutungen besitzen und X ein Halogenatom darstellt, welches man mit dem Derivat der Formel (III): 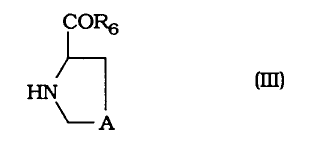 in der R₆ die bezüglich der Formel (I) angegebenen Bedeutungen besitzt, behandelt zur Bildung eines Derivats der Formel (I): 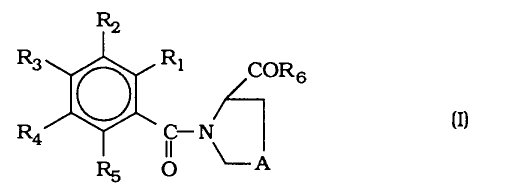 welches man gegebenenfalls in seine Isomeren auftrennt und gewünschtenfalls mit einer Säure oder gegebenenfalls mit einer pharmazeutisch annehmbaren Base in sein Salz überführt,
welches Derivat der Formel (I), wenn R₆ eine Gruppe OH darstellt, gegebenenfalls nach der eventuellen Aktivierung der Carbonsäurefunktion
- entweder mit einem Amin der Formel NR₇R₈ oder einem Derivat der Formel X-B-NR₇R₈, worin X ein Halogenatom darstellt, und B, R₇ und R₈ die bezüglich der Formel (I) angegebenen Bedeutungen besitzen, oder mit einem niedrigen aliphatischen Alkohol R'₆OH, worin R'₆ eine Niedrigalkylgruppe darstellt, umsetzt zur Bildung einer Verbindung der Formel (I), worin R₆ eine Gruppe NR₇R₈ bzw. eine Niedrigalkylgruppe darstellt,
- oder mit Imidazol behandelt, so daß man ein Derivat der Formel (I') erhält, welches Derivat der Formel (I) oder (I') man gewünschtenfalls reinigt, gegebenenfalls in seine Isomeren auftrennt und gewünschtenfalls mit einer pharmazeutisch annehmbaren Säure oder Base in ein Salz überführt.

2. Verfahren zur Herstellung der Derivate der Formel (I), worin A eine Gruppe C=O darstellt, **dadurch gekennzeichnet**, daß man ein Derivat der Formel (I/B): 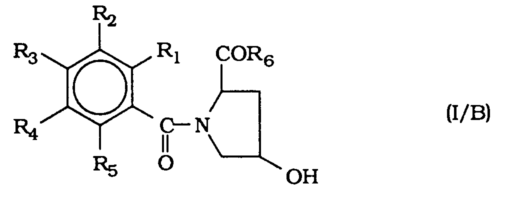 einem Sonderfall der Derivate der Formel (I), worin R₁, R₂, R₃, R₄, R₅ und R₆ die bezüglich der Formel (I) angegebenen Bedeutungen besitzen und A eine Gruppe CHOH darstellt,
mit einem Oxidationsmittel behandelt, so daß man ein Derivat der Formel (I/C) erhält: 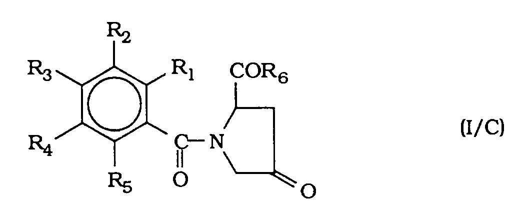 in der R₁, R₂, R₃, R₄, R₅ und R₆ die bezüglich der Formel (I) angegebenen Bedeutungen besitzen,
welches man reinigt und gewünschtenfalls je nachdem mit einer pharmazeutisch annehmbaren Säure oder Base in ein Salz überführt,
und welches man, wenn R₆ eine Hydroxylgruppe darstellt, in Abhängigkeit von dem Derivat der Formel (I), welches man herzustellen wünscht, entweder mit einem Amin der Formel NR₇R₈ oder einem Derivat X-B-NR₇R₈, worin B, R₇ und R₈ die bezüglich der Formel (I) angegebenen Bedeutungen besitzen und X ein Halogenatom darstellt, oder mit einem niedrigaliphatischen Alkohol R'₆OH, worin R'₆ eine Niedrigalkylgruppe bedeutet, behandeln kann, so daß man eine Verbindung der Formel (I) erhält, worin R₆ eine Gruppe NR₇R₈ bzw. eine Niedrigalkylgruppe darstellt,
welche man erforderlichenfalls reinigt und gegebenenfalls in die Isomeren auftrennt und man gewünschtenfalls je nachdem mit einer pharmazeutisch annehmbaren Säure oder Base in ein Salz überführt.

3. Verfahren nach Anspruch 1 zur Herstellung von Verbindungen der Formel (I), worin A eine Gruppe CHOH darstellt, von deren trans-(L)- oder (2S,4R)-Isomeren, trans-(D)- oder (2R,4S)-Isomeren, cis-(L)- oder (2S,4S)-Isomeren oder cis-(D)- oder (2R,4R)-Isomeren sowie gegebenenfalls von deren Additionssalzen mit einer pharmazeutisch annehmbaren Säure oder Base.

4. Verfahren nach Anspruch 1 zur Herstellung von Verbindungen der Formel (I), worin A eine Gruppe CHOH darstellt, der Konfiguration trans-(L) oder (2S,4R) und gegebenenfalls von deren Additionssalzen mit einer pharmazeutisch annehmbaren Säure oder Base.

5. Verfahren nach Anspruch 1 zur Herstellung von Verbindungen der Formel (I), **dadurch gekennzeichnet**, daß R₂, R₃ und R₄, die gleichartig oder verschieden sein können, jeweils eine Niedrigalkoxygruppe oder ein Wasserstoffatom oder eine Hydroxylgruppe bedeuten, während R₁ und R₅ jeweils ein Wasserstoffatom darstellen, von deren Isomeren sowie deren Additionssalzen mit einer pharmazeutisch annehmbaren Säure oder Base.

6. Verfahren nach Anspruch 1 zur Herstellung von Verbindungen der Formel (I), worin R₆ eine Hydroxylgruppe oder eine Niedrigalkoxygruppe oder eine Aminogruppe oder eine N,N-Niedrigdialkylaminogruppe oder eine 4-(2,3,4-Trimethoxy-benzyl)-piperazin-1-yl-gruppe bedeutet, von deren Isomeren sowie gegebenenfalls von deren Additionssalzen mit einer pharmazeutisch annehmbaren Säure oder Base.

7. Verfahren nach Anspruch 1 zur Herstellung von Verbindungen der Formel (I), nämlich trans-(L)-1-(3,4-Dimethoxy-benzoyl)-4-hydroxypyrrolidin-2-N',N'-diethylcarboxamid, dessen entsprechender Carbonsäure und von deren trans-(D)-, cis-(L)- und cis(D)-Isomeren sowie von deren Additionssalzen mit einer pharmazeutisch annehmbaren Base.

8. Verfahren nach Anspruch 1 zur Herstellung von Verbindungen der Formel (I), nämlich trans-(L)-1-(3,4,5-Trimethoxy-benzoyl)-4-hydroxy-pyrrolidin-2-carbonsäure, von deren trans-(D)-, cis-(L)- und cis-(D)-Isomeren sowie von deren Additionssalzen mit einer pharmazeutisch annehmbaren Base.

9. Verfahren nach Anspruch 1 zur Herstellung von Verbindungen der Formel (I), nämlich trans-(L)-1-(3,4-Dimethoxy-benzoyl)-4-hydroxy-pyrrolidin-2-N'-(cyclohexylmethyl)-carboxamld und von dessen trans-(D)-, cis-(L)- und cis-(D)-Isomeren.

10. Verfahren nach Anspruch 1 zur Herstellung von Verbindungen der Formel (I), nämlich trans-(L)-1-(3,4-Dimethoxy-benzoyl)-4-hydroxy-pyrrolidin-2-N'-cyclopropylcarboxamid und von dessen trans-(D)-, cis-(L)- und cis-(D)-Isomeren.

11. Verfahren nach Anspruch 1 zur Herstellung von Verbindungen der Formel (I), nämlich trans-(L)-1-(3,4-Methylen-dioxy benzoyl)-4-hydroxy-pyrrolidin-2-N'-(cyclohexylmethyl)-carboxamid und von dessen trans-(D)-, cis-(L)- und cis-(D)-Isomeren.

12. Verfahren nach Anspruch 1 zur Herstellung von Verbindungen der Formel (I), nämlich trans-(L)-1-(3,4-Methylendioxybenzoyl)-4-hydroxy-pyrrolidin-2-carbonsäure-3-(N',N'-dimethylamino)-propylester und von dessen trans-(D)-, cis-(L)- und cis-(D)-Isomeren sowie von deren Additionssalzen mit einer pharmazeutisch annehmbaren Säure.

13. Verfahren nach Anspruch 1 zur Herstellung von Verbindungen der Formel (I), nämlich trans-(L)-1-(3,4-Methylendioxybenzoyl)-4-hydroxy-pyrrolidin-2-carbonsäure-2-morpholinoethylester und von dessen trans-(D)-, cis-(L)- und cis-(D)-Isomeren sowie von dessen Additionssalzen mit einer pharmazeutisch annehmbaren Säure.

14. Verfahren nach Anspruch 1 zur Herstellung von Verbindungen der Formel (I), nämlich trans-(L)-1-(3,4-Dimethoxybenzoyl)-4-hydroxy-pyrrolidin-2-carbonsäure-2-morpholinoethylester und von dessen trans-(D)-, cis-(L)- und cis-(D)-Isomeren sowie von deren Additionssalzen mit einer pharmazeutisch annehmbaren Säure.

15. Verfahren nach Anspruch 1 zur Herstellung von Verbindungen der Formel (I), nämlich trans-(L)-[4-Hydroxy-1-(3,4-methylendioxybenzoyl)-pyrrolidin-2-yl]-[4-(2,3,4-trimethoxybenzyl)piperazin-1-yl)-keton und von dessen trans-(D)-, cis-(L)- und cis-(D)-Isomeren sowie von deren Additionssalzen mit einer pharmazeutisch annehmbaren Säure.

16. Verfahren nach Anspruch 1 zur Herstellung von Verbindungen der Formel (I), nämlich trans-(L)-[4-Hydroxy-1-(3,4-Dimethoxybenzoyl)-pyrrolidin-2-yl]-[4-(2,3,4-trimethoxybenzyl)-piperazin-1-yl)-keton und von dessen trans-(D)-, cis-(L)- und cis-(D)-Isomeren sowie von deren Additionssalzen mit einer pharmazeutisch annehmbaren Säure.

17. Verfahren nach Anspruch 1 zur Herstellung von Verbindungen der Formel (I), worin R₆ eine Niedrigalkoxygruppe darstellt sowie von deren Isomeren.

18. Verfahren nach Anspruch 1 zur Herstellung von Verbindungen der Formel (I), nämlich trans-(L)-1-(3,4,5-Trimethoxybenzoyl)-4-hydroxy-pyrrolidin-2-N'-cyclohexylmethylcarboxamid und von dessen trans-(D)-, cis-(L)- und cis-(D)-Isomeren.

19. Verfahren nach Anspruch 1 zur Herstellung von Verbindungen der Formel (I), nämlich trans-(L)-1-(3,4-Dimethoxybenzoyl)-4-hydroxypyrrolidin-2-N'-cyclooctylcarboxamid und von dessen trans-(D)-, cis-(L)- und cis-(D)-Isomeren.

20. Verfahren nach Anspruch 1 zur Herstellung von Verbindungen der Formel (I), nämlich trans-(L)-1-(3,4-Dimethoxybenzoyl)-4,hydroxy-pyrrolidin-2-carboxamid und von dessen trans-(D)-, cis-(L)- und cis-(D)-Isomeren.

21. Verfahren nach Anspruch 1 zur Herstellung von Verbindungen der Formel (I), nämlich trans-(L)-1-(3,4,5-Trimethoxybenzoyl)-4-hydroxy-pyrrolidin-2-carbonsäureethylester und von dessen trans-(D)-, cis-(L)- und cis-(D)-Isomeren.

22. Verfahren zur Herstellung einer pharmazeutischen Zubereitung, die als Wirkstoff mindestens eine Verbindung, erhalten nach einem der Ansprüche 1 bis 21 enthält in Kombination mit einem oder mehreren inerten, nichttoxischen, pharmazeutische annehmbaren Trägermaterialien oder Bindemitteln.

23. Verfahren nach Anspruch 22 zur Herstellung einer pharmazeutischen Zubereitung enthaltend mindestens einen Wirkstoff nach einem der Ansprüche 1 bis 21 zur Verwendung bei der Behandlung von Erkrankungen, die sich durch ischämische hypoxische Störungen oder Sauerstoffversorgungsstörungen ergeben, Syndromen von intellektuellem Defizit, pathologischen Zuständen des Alterns, Aufmerksamkeitsstörungen, konstituierten Zerebralinfarkten und Schwindelzuständen zentralen Ursprungs geeignet sind.
